(19) Europäisches Patentamt
European Patent Office
Office européen des brevets

(11) Veröffentlichungsnummer: **0 464 572 A2**

(12) # EUROPÄISCHE PATENTANMELDUNG

(21) Anmeldenummer: **91110400.8**

(22) Anmeldetag: **24.06.91**

(51) Int. Cl.5: **C07D 487/04**, C07D 471/04, A61K 31/495, A61K 31/435, //(C07D487/04,249:00,237:00), (C07D487/04,249:00,241:00)

(30) Priorität: **28.06.90 CH 2159/90**

(43) Veröffentlichungstag der Anmeldung: **08.01.92 Patentblatt 92/02**

(84) Benannte Vertragsstaaten: **AT BE CH DE DK ES FR GB GR IT LI LU NL SE**

(71) Anmelder: **F. HOFFMANN-LA ROCHE AG**
**Postfach 3255**
**CH-4002 Basel(CH)**

(72) Erfinder: **Branca, Ouirico, Dr.**
**Lenzgasse 2**
**CH-4056 Basel(CH)**
Erfinder: **Heitz, Marie-Paule, Dr.**
**122 rue de Mulhouse**
**F-68300 St. Louis(FR)**
Erfinder: **Müller, Marcel, Dr.**
**Ouellenweg 10**
**CH-4402 Frenkendorf(CH)**
Erfinder: **Neidhart, Werner, Dr.**
**5, rue du Jura**
**F-68870 Bartenheim(FR)**
Erfinder: **Stadler, Heinz, Dr.**
**Waldhofstrasse 37**
**CH-4310 Rheinfelden(CH)**
Erfinder: **Vieira, Eric, Dr.**
**Frobenstrasse 57**
**CH-4053 Basel(CH)**
Erfinder: **Wostl, Wolfgang, Dr.**
**Im Strick 2**
**W-7889 Grenzach-Wyhlen(DE)**

(74) Vertreter: **Lederer, Franz, Dr. et al**
**Patentanwalt Dr. Franz Lederer**
**Lucile-Grahn-Strasse 22**
**W-8000 München 80(DE)**

(54) **Aminosäurederivate.**

(57) Die Verbindungen der Formel

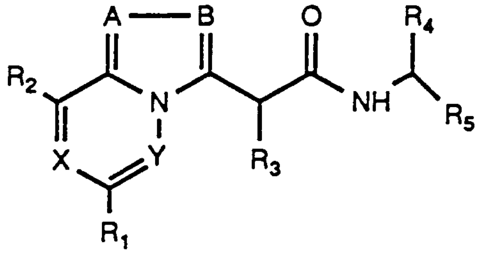

I

worin A, B, X, Y, $R^1$, $R^2$, $R^3$, $R^4$ und $R^5$ die in Anspruch 1 angegebene Bedeutung besitzen, in Form von optisch reinen Diastereomeren, Diastereomerengemischen, diastereomeren Racematen oder Gemischen von diastereomeren Racematen sowie pharmazeutisch verwendbare Salze davon hemmen die Wirkung des natürlichen Enzyms Renin und können demnach in Form pharmazeutischer Präparate bei der Bekämpfung bzw. Verhütung von Bluthochdruck und Herzinsuffizienz verwendet werden. Sie können nach verschiedenen, an sich bekannten Verfahren hergestellt werden.

EP 0 464 572 A2

Die vorliegende Erfindung betrifft Aminosäurederivate. Im speziellen betrifft sie Aminosäurederivate der allgemeinen Formel

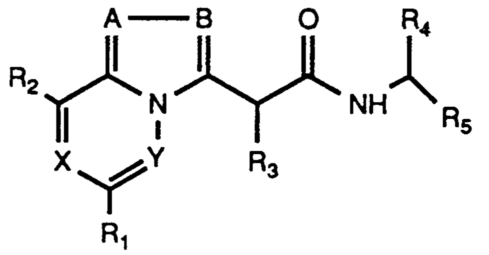

I

worin einer von A und B ein Stickstoffatom und der andere -CH- oder beide ein Stickstoffatom, einer von X und Y ein Stickstoffatom und der andere -CH- oder beide -CH-, $R^1$ Phenyl, Pyridyl oder Thienyl, $R^2$ Alkyl oder Arylalkyl, $R^3$ Wasserstoff, Alkyl, Imidazol-2-ylmethyl, Imidazol-4-ylmethyl, Pyridylmethyl, Pyrazol-3-ylmethyl, Thien-2-ylmethyl, Thiazol-4-ylmethyl, Alkylthiomethyl, Carbamoylmethyl, Carbamoyläthyl oder Benzyl, $R^4$ Cyclohexylmethyl, Benzyl oder Isobutyl und $R^5$ eine der Gruppen

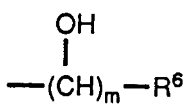

(a) und

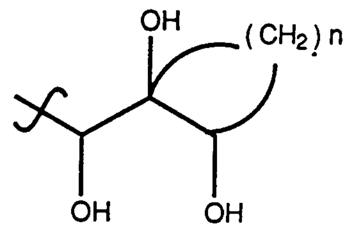

(b)

bedeuten, worin $R^6$ Cycloalkyl, Alkyl, Alkenyl oder Arylalkyl, m die Zahl 2 oder 3 und n die Zahl 3 oder 4 bedeuten,
in Form von optisch reinen Diastereomeren,
Diastereomerengemischen, diastereomeren Racematen oder Gemischen von diastereomeren Racematen sowie pharmazeutisch verwendbare Salze dieser Verbindungen.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimittel, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

Der in der vorliegenden Beschreibung verwendete Ausdruck "Alkyl" - allein oder in Kombination - bedeutet geradkettige und verzweigte, gesättigte Kohlenwasserstoffreste mit 1-8, vorzugsweise 1-4, Kohlenstoffatomen, wie Methyl, Aethyl, n-Propyl, Isopropyl, n-Butyl, Isobutyl, sec.-Butyl, t-Butyl, Pentyl, Hexyl und dergleichen. Der Ausdruck "Alkoxy" bedeutet Alkyläthergruppen, worin der Ausdruck "Alkyl" die obige Bedeutung hat, wie Methoxy, Aethoxy, Propoxy, Isopropoxy, Butoxy, Isobutoxy, sec.-Butoxy, t-Butoxy und dergleichen. Der Ausdruck "Cycloalkyl" bedeutet gesättigte, cyclische Kohlenwasserstoffreste mit 3-8, vorzugsweise 3-6, Kohlenstoffatomen, wie Cyclopropyl, Cyclobutyl, Cyclopentyl, Cyclohexyl und dergleichen. Der Ausdruck "Alkenyl" betrifft geradkettige und verzweigte, ungesättigte Kohlenwasserstoffreste mit 2-8, vorzugsweise 2-4, Kohlenstoffatomen, wie Vinyl, Allyl, 2-Butenyl, 3-Butenyl und dergleichen. Der Ausdruck "Alkanoyloxy" bedeutet den über ein Sauerstoffatom gebundenen Säurerest einer geradkettigen oder verzweigten Alkansäure mit 1-8, vorzugsweise 1-4, Kohlenstoffatomen, wie Formyloxy, Acetyloxy, Propionyloxy, Butyryloxy, Valeryloxy, Isovaleryloxy und dergleichen. Der Ausdruck "Arylalkyl" bezeichnet geradkettige oder verzweigte Alkylgruppen, worin ein oder mehrere Wasserstoffatome durch Arylgruppen ersetzt sind, wobei der Ausdruck "Aryl" einen gegebenenfalls durch Alkyl, Alkoxy, Alkanoyloxy, Amino, Alkylamino, Dialkylamino, Alkylcarbonylamino, Hydroxy, Halogen, Trifluormethyl oder Nitro ein- oder mehrfach substituierten mono- oder bicyclischen aromatischen Kohlenwasserstoffrest mit 6-14 Kohlenstoffatomen, wie Phenyl, α- oder β-Naphthyl, Indenyl, Anthryl oder Phenanthryl und dergleichen bedeutet. Beispiele von Arylalkylgruppen sind Benzyl, Diphenylmethyl, Trityl, α- oder β-Naphthylmethyl, 2-Phenyläthyl, 3-Phenyl-2-propyl, 4-Phenyl-3-butyl, 2-(α- oder β-Naphthyl)äthyl, 3-α-Naphthyl-2-propyl, 4-α-Naphthyl-3-butyl und dergleichen, wobei der aromatische Rest jeweils wie oben angegeben ein- oder mehrfach substituiert sein kann.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens drei asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Bevorzugt sind solche Verbindungen der Formel I, worin A und B je ein Stickstoffatom bedeuten. X bedeutet vorzugsweise ein Stickstoffatom und Y -CH-. Bevorzugt sind auch Verbindungen der Formel I, worin $R^1$ Pyridyl, besonders bevorzugt 3-Pyridyl, bedeutet. $R^2$ bedeutet vorzugsweise Alkyl oder Phenylalkyl, besonders bevorzugt Propyl, Isopropyl, Isobutyl, 1-Methylpropyl oder Benzyl. Die bevorzugte Bedeutung von $R^3$ ist Wasserstoff, Imidazol-2-ylmethyl, Imidazol-4-ylmethyl oder Pyridylmethyl, besonders bevorzugt Imidazol-4-ylmethyl oder Pyridyl-3-methyl. Bevorzugt ist die Bedeutung Cyclohexylmethyl für $R^4$. $R^5$ bedeutet vorzugsweise die Gruppe (a). Die bevorzugte Bedeutung für m ist die Zahl 2 und für $R^6$ Cycloalkyl.

Aus dem obigen folgt, dass solche Verbindungen der Formel I ganz besonders bevorzugt sind, worin A, B und X je ein Stickstoffatom, Y -CH-, $R^1$ 3-Pyridyl, $R^2$ Propyl, Isopropyl, Isobutyl, 1-Methylpropyl oder Benzyl, $R^3$ Imidazol-4-methyl oder Pyridyl-3-methyl, $R^4$ Cyclohexylmethyl und $R^5$ die Gruppe (a) bedeuten, worin m die Zahl 2 und $R^6$ Cycloalkyl bedeuten.

Ganz speziell bevorzugte Verbindungen der Formel I sind:

(R oder S)-N-[(1S,2R,3S)-1-(Cyclohexylmethyl) -3-cyclopropyl-2,3-dihydroxypropyl] -8-propyl-α,6-bis(3-pyridyl)-s-triazolo[4,3-a]pyrazin -3-acetamid,

(R oder S)-N-[(1S,2R,3S)-1-(Cyclohexylmethyl)-3-cyclopropyl -2,3-dihydroxypropyl]-8-isopropyl-α-(imidazol -4-ylmethyl)-6-(3-pyridyl)-s-triazolo[4,3-a]pyrazin -3-acetamid,

(R oder S)-N-[(1S,2R,3S)-1-(Cyclohexylmethyl)-3-cyclohexyl -2,3-dihydroxypropyl]-α-(imidazol-4-ylmethyl) -8-isobutyl-6-(3-pyridyl)-s-triazolo[4,3-a]pyrazin -3-acetat,

(R oder S)-N-[(1S,2R,3S)-1-(Cyclohexylmethyl)-2,3-dihydroxy -5-methylhexyl]-8-propyl-6-(3-pyridyl) -α-(3-pyridylmethyl)-s-triazolo[4,3-a]pyrazin -3-acetamid,

(S oder R)-N-[(1S,2R,3S)-1-(Cyclohexylmethyl)-3-cyclopropyl -2,3-dihydroxypropyl]-8-[(RS)-1-methylpropyl] -α-(3-pyridylmethyl)-6-(3-pyridyl) -s-triazolo[4,3-a]pyrazin-3-acetamid und

N-[(1S,2R,3S)-1-(Cyclohexylmethyl-3-cyclopropyl -2,3-dihydroxypropyl]-8-[(RS)-1-methylpropyl] -6-(3-pyridyl)-s-triazolo[4,3-a]pyrazin-3-carboxamid.

Die Verbindungen der Formel I in Form von optisch reinen Diastereomeren, Diastereomerengemischen, diastereomeren Racematen oder Gemischen von diastereomeren Racematen sowie pharmazeutisch verwendbare Salze davon können hergestellt werden, indem man

a) eine Verbindung der allgemeinen Formel

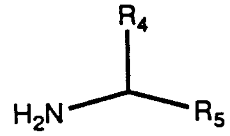

II

worin $R^4$ und $R^5$ die oben angegebene Bedeutung besitzen,

mit einer Verbindung der allgemeinen Formel

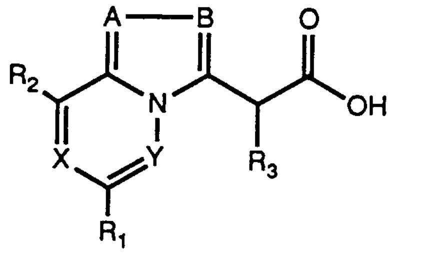

III

worin A, B, X, Y, $R^1$, $R^2$ und $R^3$ die oben angegebene Bedeutung besitzen,
oder einem aktivierten Derivat davon umsetzt, und
b) erwünschtenfalls ein Gemisch diastereomerer Racemate in die diastereomeren Racemate oder optisch reinen Diastereomeren auftrennt, und/oder
c) erwünschtenfalls ein Diastereomerengemisch in die optisch reinen Diastereomeren auftrennt, und/oder
d) erwünschtenfalls eine erhaltene Verbindung in ein pharmazeutisch verwendbares Salz überführt.

Die Acylierung einer Verbindung der Formel II erfolgt nach an sich bekannten Methoden. Geeignete Acylierungsmittel sind insbesondere aktivierte Säurederivate, wie Ester, gemischte Ester, Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0° C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage. Die Reaktion erfolgt unter in der Peptidchemie üblichen Reaktionsbedingungen, d.h. vorzugsweise in Gegenwart eines Kondensationsmittels wie HBTU (O-Benzotriazolyl-N,N,N',N'-tetramethyluronium-hexafluorophosphat), BOP (Benzotriazol-1-yloxy-tris-(dimethylamino)phosphonium-hexafluorophosphat),BOPC (Bis(2-oxo-2-oxozolidinyl)phosphinchlorid), HOBT (N-Hydroxybenzotriazol), DBU (1,8-Diazabicyclo[5.4.O]undec-7-en),DCC (Dicyclohexylcarbodiimid), EDC (N-Aethyl-N'(3-dimethylaminopropyl)carbodiimid-hydrochlorid), Hünigbase (Aethyldiisopropylamin), und dergleichen. Die Reaktion wird zweckmässigerweise in einem unter den Reaktionsbedingungen inerten organischen Lösungsmittel oder Lösungsmittelgemisch bei einer Temperatur zwischen etwa 0° und 50° C, vorzugsweise bei etwa Raumtemperatur durchgeführt. Als Lösungsmittel kommen insbesondere Dimethylformamid, Methylenchlorid, Acetonitril, Tetrahydrofuran, und dergleichen in Frage.

Die Ausgangsstoffe der Formel II, worin $R^5$ die Gruppe (a), worin m die Zahl 3 bedeutet, oder die Gruppe (b) bedeutet, sind neu und ebenfalls Gegenstand der vorliegenden Erfindung. Sie können beispielsweise hergestellt werden, indem man in einer Verbindung der allgemeinen Formeln

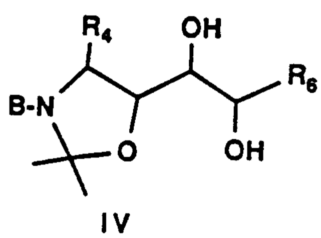

IV

und

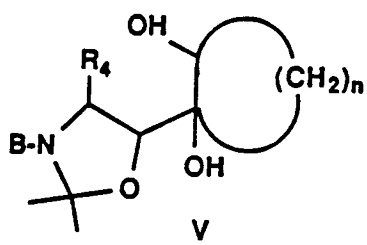

V

worin B eine Aminoschutzgruppe, vorzugsweise t-Butoxycarbonyl oder Benzyloxycarbonyl, bedeutet und $R^4$, $R^6$ und n die oben angegebene Bedeutung besitzen,
die Aminoschutzgruppe und gleichzeitig auch die O-Schutzgruppe abspaltet.

Die Abspaltung der N-Schutzgruppe und O-Schutzgruppe erfolgt ebenfalls nach an sich bekannten Methoden, beispielsweise in einem unter den Reaktionsbedingungen inerten organischen Lösungsmittel oder Lösungsmittelgemisch bei einer Temperatur zwischen etwa 0° C und der Raumtemperatur mit einer Säure, wie Chlorwasserstoffsäure, Trifluoressigsäure, und dergleichen. Geeignete Lösungsmittel sind Aether, wie Tetrahydrofuran oder Dioxan, Alkohole, wie Methanol, oder chlorierte Kohlenwasserstoffe, wie Methylenchlorid, und dergleichen. Unter diesen Reaktionsbedingungen wird - wie bereits erwähnt - gleichzeitig der Oxazolidinring aufgespalten.

Die Ausgangsstoffe der Formel II, worin $R^5$ die Gruppe (a) bedeutet, worin m die Zahl 2 bedeutet, sind

bekannt oder können in Analogie zur Herstellung der bekannten Verbindungen erhalten werden.

Die Verbindungen der Formeln IV und V sind ebenfalls neu und Gegenstand vorliegender Erfindung. Sie können nach verschiedenen, an sich bekannten Methoden ausgehend von Verbindungen der Formel VI hergestellt werden. Diese Herstellungsverfahren sind im nachfolgenden Schema I zusammengefasst. In diesem Schema bedeutet Met ein Metall, wie Lithium oder Magnesium, und besitzen B, $R^4$, $R^6$ und n die oben angegebene Bedeutung. Bezüglich der genauen Reaktionsbedingungen wird auf den Beispielteil verwiesen.

**Schema I**

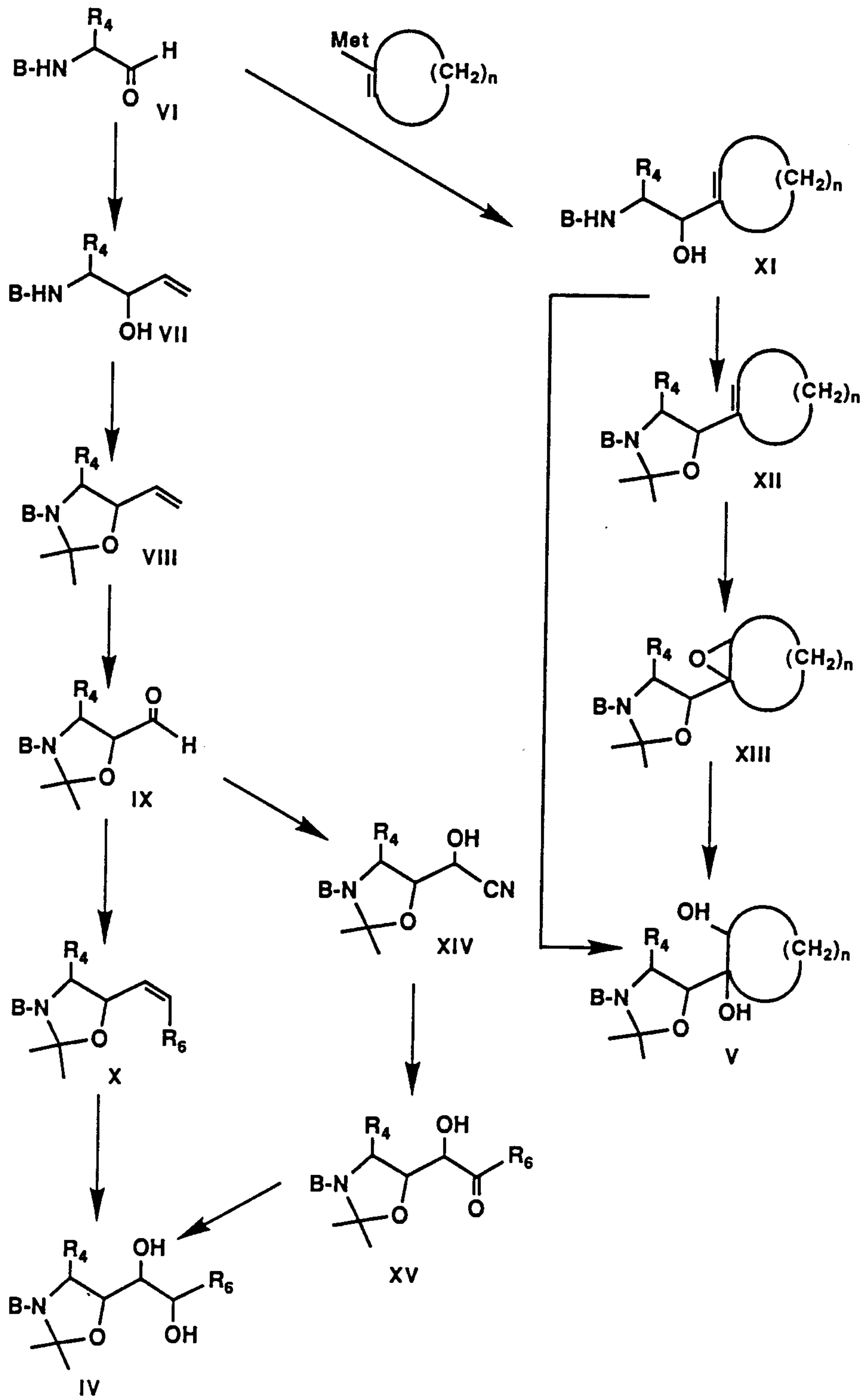

Die Ausgangsstoffe der Formel III, worin A, B und X je ein Stickstoffatom und Y -CH- bedeuten, sind aus EP-A 0.369.743 bekannt oder können in Analogie zur Herstellung der bekannten Verbindungen erhalten werden.

Die übrigen Verbindungen der Formel III sind ebenfalls neu und Gegenstand vorliegender Erfindung. Sie können nach verschiedenen, an sich bekannten, teilweise zum in EP-A 0.369.743 beschriebenen

Verfahren analogen Methoden ausgehend von entsprechenden Pyrazinonen, Pyridazinonen und Pyridinonen hergestellt werden. Diese Herstellungsverfahren sowie das Verfahren gemäss EP-A 0.369.743 sind in den nachfolgenden Schemata II-IV zusammengefasst. In diesen Schemata bedeutet R Alkyl und besitzen A, B, R,$^1$, $R^2$ und $R^3$ die oben angegebene Bedeutung. Die Pyrazinone der Formel XVI in Schema II und die Pyridazinone der Formel XXIV in Schema III sowie deren Herstellung sind teilweise in EPA 0,369,743 bzw. Chim. Ther., 6, 109 (1971) beschrieben oder können in Analogie zur Herstellung der bekannten Verbindungen erhalten werden. Die Pyridinone der Formel XXXII in Schema IV sind neu, gehören aber einer bekannten Stoffklasse an. Sie können in Analogie zur Herstellung der in Tetrahedron Letters 1974, 1183 und Arch. Pharm., 317, 183 (1984) beschriebenen Verbindungen hergestellt werden.

## Schema II

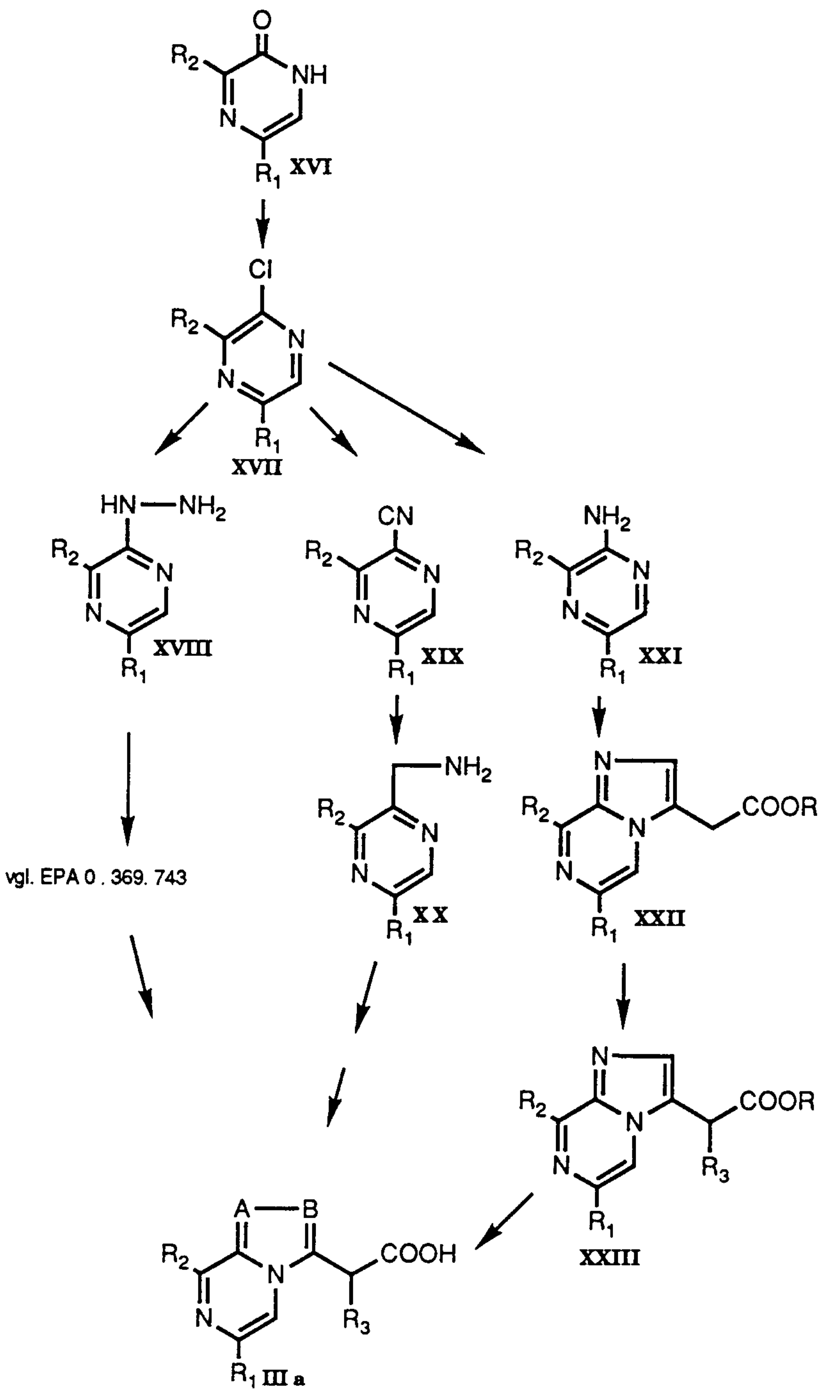

XVI
XVII
XVIII
XIX
XXI
XX
XXII
XXIII
IIIa

## Schema III

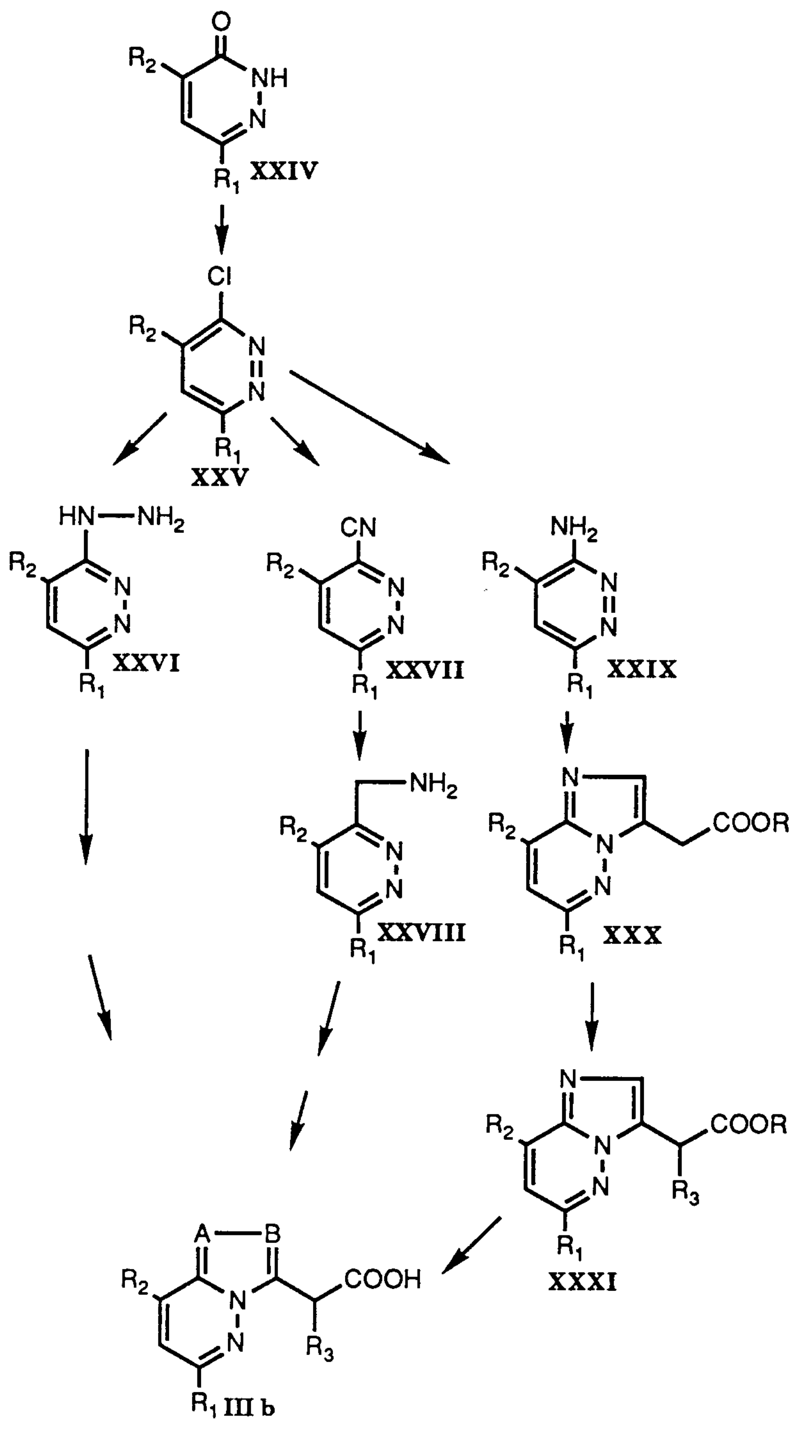

XXIV

XXV

XXVI

XXVII

XXIX

XXVIII

XXX

XXXI

III b

**Schema IV**

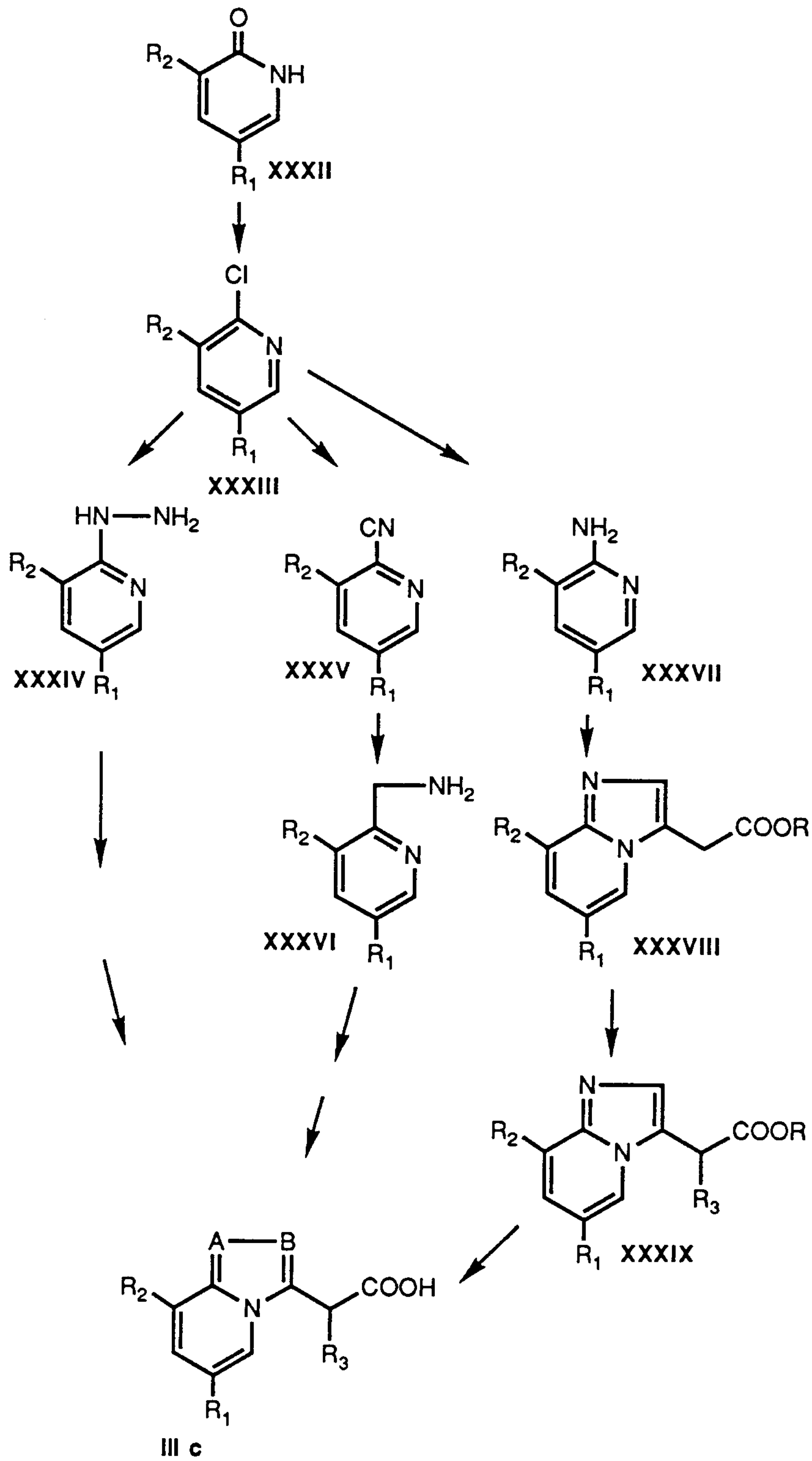

XXXII

XXXIII

XXXIV

XXXV

XXXVII

XXXVI

XXXVIII

XXXIX

III c

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des

daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.

Die Wirkung von Renin-Hemmern kann experimentell mittels des nachstehend beschriebenen In vitro-Tests gezeigt werden:

In vitro-Test mit reinem Human-Renin

Der Test wird in Eppendorf Röhrchen durchgeführt. Die Inkubationsmischung besteht aus (1) 100 µl Human-Renin in Puffer A (0,1M Natriumphosphatlösung, pH 7,4, enthaltend 0,1% Rinderserumalbumin, 0,1 % Natriumazid und 1 mM Aethylendiamintetraessigsäure),genügend für eine Renin-Aktivität von 2-3 ng Angiotensin I/ ml/Std.; (2) 145 µl Puffer A; (3) 30 µl von 10 µM humanem Tetradekapeptid-Renin-substrat (hTD) in 10 mM Salzsäure; (4) 15 µl Dimethylsulfoxid mit bzw. ohne Hemmer und (5) 10 µl einer 0,03 molaren Lösung von Hydroxychinolinsulfat in Wasser.

Die Proben werden drei Stunden bei 37°C bzw. 4°C in Triplikaten inkubiert. 2 x 100 µl Proben pro Versuchsröhrchen werden dann verwendet, um die Produktion von Angiotensin I via RIA (standard radioimmunoassay; Clinical Assay solid phase kit) zu messen. Kreuzreaktivitäten der verwendeten Antikörper im RIA sind: Angiotensin I 100 %; Angiotensin II 0,0013 %; hTD (Angiotensin I-Val-Ile-His-Ser-OH) 0,09 %. Die Produktion von Angiotensin I wird durch die Differenz zwischen dem Versuch bei 37°C und demjenigen bei 4°C bestimmt.

Folgende Kontrollen werden mitgeführt:

(a) Inkubation von hTD-Proben ohne Renin und ohne Hemmer bei 37°C und 4°C. Die Differenz zwischen diesen beiden Werten ergibt den Grundwert der Angiotensin I-Produktion.

(b) Inkubation von hTD-Proben mit Renin, jedoch ohne Hemmer bei 37°C und 4°C. Die Differenz zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse (=100%) durch Renin.

Die Resultate werden als $IC_{50}$-Werte angegeben, welche diejenige Konzentration des Hemmers bezeichnen, bei welcher die enzymatische Aktivität um 50% gehemmt wird. Die $IC_{50}$-Werte werden aus einer linearen Regressionskurve aus einem logit-log plot ermittelt.

Die in diesem Test erhaltenen Resultate sind in der folgenden Tabelle zusammengefasst:

Tabelle

| Verbindung | $IC_{50}$-Werte in nMol/lt. |
|---|---|
| A | 61 |
| B | 43 |
| C | 42 |
| D | 57 |
| E | 48 |
| F | 11 |

A= (R oder S)-N-[(1S,2R,3S)-1-(Cyclohexylmethyl) -3-cyclopropyl-2,3-dihydroxypropyl] -8-propyl-α,6-bis(3-pyridyl)-s-triazolo[4,3-a]pyrazin -3-acetamid

B= (R oder S)-N-[(1S,2R,3S)-1-(Cyclohexylmethyl)-3-cyclopropyl -2,3-dihydroxypropyl]-8-isopropyl-α-(imidazol -4-ylmethyl)-6-(3-pyridyl)-s-triazolo[4,3-a]pyrazin -3-acetamid

C= (R oder S)-N-[(1S,2R,3S)-1-(Cyclohexylmethyl)-3-cyclohexyl -2,3-dihydroxypropyl]-α-(imidazol-4-ylmethyl) -8-isobutyl-6-(3-pyridyl)-s-triazolo[4,3-a]pyrazin -3-acetat

D= (R oder S)-N-[(1S,2R,3S)-1-(Cyclohexylmethyl)-2,3-dihydroxy -5-methylhexyl]-8-propyl-6-(3-pyridyl) -α-(3-pyridylmethyl)-s-triazolo[4,3-a]pyrazin -3-acetamid

E= (S oder R)-N-[(1S,2R,3S)-1-(Cyclohexylmethyl)-3-cyclopropyl -2,3-dihydroxypropyl]-8-[(RS)-1-methylpropyl] -α-(3-pyridylmethyl)-6-(3-pyridyl) -s-triazolo-[4,3-a]pyrazin-3-acetamid

F = N-[(1S,2R,3S)-1-(Cyclohexylmethyl-3-cyclopropyl -2,3-dihydroxypropyl]-8-[(RS)-1-methylpropyl] -6-(3-pyridyl)-s-triazolo[4,3-a]pyrazin-3-carboxamid.

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben.

Beispiel 1

218 mg (0.56 mMol) rac-8-Propyl-6-(3-pyridyl)-α-(3-pyridylmethyl) -s-triazolo[4,3-a]pyrazin-3-essigsäure, 127 mg (0.56 mMol) (1S,2R,3S)-3-Amino-4-cyclohexyl-1-cyclopropyl-1,2-butandiol (EP-A 0.332.008)), 248 mg (0.56 mMol) Benzotriazol-1-yloxy-tris-(dimethylamino)phosphoniumhexafluorophosphat (BOP) löst man in 20 ml Methylenchlorid ($CH_2Cl_2$), fügt 0.192 ml (1.12 mMol) Hünigbase zu und rührt die Lösung 12 Stunden bei Raumtemperatur. Anschliessend verteilt man zwischen $CH_2Cl_2$ und gesättigter Ammoniumchloridlösung, trocknet die organische Phase über Natriumsulfat und entfernt schliesslich das Lösungsmittel unter vermindertem Druck.

Das Rohprodukt (600 mg gelbes Oel), welches das gewünschte Produkt als ein 1:1-Gemisch zweier Epimere enthält, wird durch Chromatographie an Kieselgel mit einem 10:1-Gemisch von $CH_2Cl_2$ und Methanol gereinigt und in die beiden Epimeren aufgetrennt. Man erhält so 33 mg (10%) des polareren Epimers (R oder S)-N-[(1S,2R,3S)-1-(Cyclohexylmethyl)-3-cyclopropyl-2,3-dihydroxypropyl]-8-propyl-α,6-bis(3-pyridyl)-s-triazolo[4,3-a]pyrazin-3-acetamid als gelbe Kristalle, MS: 598 $(M+H)^+$, 76 mg (22.7%) des reinen, weniger polaren Epimers (S oder R)-N-[(1S,2R,3S)-1-(Cyclohexylmethyl)-3-cyclopropyl -2,3-dihydroxypropyl] -8-propyl-α,6-bis(3-pyridyl)-s-triazolo[4,3-a]pyrazin-3-acetamid, MS: 598 $(M+H)^+$ und 166 mg (49.6%) eines Gemisches beider Epimerer, d.h. (RS)-N-[(1S,2R,3S)-1-(Cyclohexylmethyl)-3-cyclopropyl -2,3-dihydroxypropyl]-8-propyl-α,6-bis(3-pyridyl)-s-triazolo[4,3-a]pyrazin-3-acetamid, MS: 598 $(M+H)^+$.

Die als Ausgangsstoff eingesetzte rac-8-Propyl-6-(3-pyridyl)-α-(3-pyridylmethyl) -s-triazolo[4,3-a)pyrazin-3-essigsäure wurde wie folgt hergestellt:

Man löst 3.31 g (11.7 mMol) 3-(2-Amino-1,1-diäthoxyäthyl)pyridin [Org. Synthesis 64, 19(1986)] und 1.17 ml (11.7 mMol) 2-Oxo-n-valeriansäure in 150 ml Dimethylformamid, gibt 2.63 ml (23.4 mMol) N-Methylmorpholin und 4.85 g (12.8 mMol) TBTU [2-(1H-Benzotriazol-1-yl)-1,1,3,3-tetramethyluroniumtetrafluoroborat] zu und rührt die Lösung 14 Stunden bei Raumtemperatur unter Argon. Man verteilt zwischen Essigester und Wasser und erhält nach üblicher Aufarbeitung der organischen Phase 2.6 g Rohprodukt als braunes Oel, welches durch Chromatographie an Kieselgel (Laufmittel: 95:5-Gemisch von $CH_2Cl_2$ und Aether) gereinigt wird, Ausbeute: 1.42 g (39%) N-[2,2-Diäthoxy-2-(3-pyridyl)äthyl]-2-oxovaleramid, MS: 309 $(M+H)^+$.

500 mg (1.62 mMol) N-[2,2-Diäthoxy-2-(3-pyridyl)äthyl]-2-oxovaleramid versetzt man mit 20 ml 2N Salzsäure und rührt anschliessend 22 Stunden bei 30°. Danach entfernt man das Lösungsmittel am Hochvakuum, behandelt den Rückstand zweimal mit Toluol, welches man jeweils unter vermindertem Druck wieder entfernt.

Man erhält so das gewünschte Produkt als beige Kristalle, Ausbeute: 420 mg (95%). Umkristallisation aus Methanol/Aether liefert N-(Nicotinoylmethyl)-2-oxovaleramid, Schmelzpunkt 80-82°.

150 mg (0.55 mMol) N-(Nicotinoylmethyl)-2-oxovaleramid und 1.5 g (19.3 mMol) Ammoniumacetat löst man in 10 ml Aethanol und erhitzt die Lösung 90 Minuten zum Rückfluss. Schliesslich giesst man auf 50 ml Eis/Wasser, extrahiert dreimal mit jeweils 20 ml Essigester, wäscht die organische Phase mit 30 ml Wasser, trocknet über Natriumsulfat und entfernt das Lösungsmittel schliesslich unter vermindertem Druck, Rohausbeute: 195 mg gelbe Kristalle. Umkristallisation aus Essigester liefert 3-Propyl-5-(3-pyridyl)-2(1H)-pyrazindion, Schmelzpunkt 188-189° (Zers.).

430 mg (2 mMol) 3-Propyl-5-(3-pyridyl)-2(1H)-pyrazindion versetzt man mit 2 ml Phosphoroxychlorid und erhitzt die Mischung anschliessend 5 Stunden zum Rückfluss. Schliesslich entfernt man das Phosphoroxychlorid unter vermindertem Druck, dampft den Rückstand einmal mit Toluol ein, löst das Rohprodukt in 20 ml $CH_2Cl_2$ und wäscht mit 10 ml Eiswasser. Die Wasserphase extrahiert man einmal mit 10 ml $CH_2Cl_2$ und trocknet die vereinigten organischen Phasen über Natriumsulfat. Man entfernt das Lösungsmittel unter vermindertem Druck und erhält das gewünschte 2-Chlor-3-propyl-5-(3-pyridyl)pyrazin als beige Kristalle, Ausbeute: 468 mg(36%); Schmelzpunkt 207-209° (aus $CH_2Cl_2$/Aether).

Man versetzt 2.5 ml Pyridin mit 2.5 ml (51.4 mMol) Hydrazinhydrat, fügt 500 mg (1.85 mMol) 2-Chlor-3-propyl-5-(3-pyridyl)pyrazin zu und erhitzt anschliessend 90 Minuten zum Rückfluss. Danach lässt man abkühlen, gibt 10 ml Wasser zur Reaktionslösung, kühlt die Mischung 30 Minuten bei 5° und nutscht schliesslich das auskristallisierte Produkt ab.

Das Rohprodukt wird unter vermindertem Druck bei 40° über Kaliumhydroxyd getrocknet und aus Alkohol/Wasser (1:1) umkristallisiert, wobei man 270 mg (63.6%) 2-Hydrazino-3-propyl-5-(3-pyridyl)pyrazin als beige Nadeln erhält, Schmelzpunkt 162-163°.

340 mg (1.35 mMol) Diäthyl (3-pyridyl)malonat (Arch. Pharm. 308, (1975) 663), 311 mg (1.35 mMol) 2-Hydrazino-3-propyl-5-(3-pyridyl)pyrazin und 257 mg (1.35 mMol) p-Toluolsulfonsäure versetzt man mit Xylol (150 ml) und erhitzt die Mischung anschliessend 12 Stunden zum Rückfluss am Wasserabscheider. Man destilliert danach das Xylol ab, löst den Rückstand in $CH_2Cl_2$, wäscht mit Natriumbicarbonatlösung, trocknet die organische Phase über Natriumsulfat und entfernt das Lösungsmittel unter vermindertem Druck. Der Rückstand wird an Kieselgel mit einem 10:1-Gemisch von $CH_2Cl_2$ und Methanol chromatographiert, wobei man 300 mg (53%) Aethyl rac-8-propyl-6-(3-pyridyl)-α-(3-pyridylmethyl) -s-triazolo[4,3-a]pyrazin-3-acetat, MS: 416 $(M)^+$, erhält.

300 mg (0.72 mMol) Aethyl rac-8-propyl-6-(3-pyridyl)-α-(3-pyridylmethyl) -s-triazolo[4,3-a]pyrazin-3-acetat löst man in 50 ml Aethanol, versetzt mit 1.44 ml 1N Natronlauge (1.44 mMol) und erhitzt die Lösung anschliessend 2 Stunden auf 50°. Man neutralisiert durch Zugabe von 1.44 ml 1N Salzsäure (pH 5) und dampft unter vermindertem Druck zur Trockene ein. Schliesslich verteilt man zwischen Wasser (5 ml) und einem 10:1-Gemisch von $CH_2Cl_2$ und Methanol (50 ml). Die organische Phase trocknet man über Natriumsulfat und entfernt das Lösungsmittel unter vermindertem Druck. So erhält man 300 mg (53%) rac-8-Propyl-6-(3-pyridyl)-α-(3-pyridylmethyl) -s-triazolo[4,3-a]pyrazin-3-essigsäure als gelbes amorphes Pulver, $R_F$:0.1 ($CH_2Cl_2$/MeOH; 5:1).

Beispiel 2

In analoger Weise wie in Beispiel 1 beschrieben wurden die folgenden Verbindungen hergestellt:

- Aus rac-α-(Imidazol-4-ylmethyl)-8-propyl-6-(3-pyridyl)-s-triazolo[4,3-a]pyrazin-3-essigsäure und (1S,2R,3S)-3-Amino-4-cyclohexyl-1-cyclopropyl-1,2-butandiol die beiden Epimeren (R oder S)-N-[-

(1S,2R,3S)-1-(Cyclohexylmethyl)-3-cyclopropyl -2,3-dihydroxypropyl]-α-imidazol-4-yl-8-propyl -6-(3-pyridyl)-s-triazolo[4,3-a]pyrazin-3-acetamid und (R oder S)-N-[(1S,2R,3S)-1-(Cyclohexylmethyl)-3-cyclopropyl -2-3-dihydroxypropyl]-α-(imidazol-4-ylmethyl)-8-propyl -6-(3-pyridyl)-s-triazolo[4,3-a]pyrazin-3-acetamid als jeweils hellgelbe Festkörper, MS: 587 $(M+H)^+$;

- aus 8-Propyl-6-(3-pyridyl)-s-triazolo[4,3-a]pyrazin -3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl -2,3-dihydroxypropyl]-8-propyl-6-(3-pyridyl) -s-triazolo[4,3-a]pyrazin-3-acetamid als Festkörper, MS: 507 $(M+H)^+$;
- aus rac-8-Isopropyl-6-(3-pyridyl)-α-(3-pyridylmethyl) -s-triazolo[4,3-a]pyrazin-3-essigsäure und (1S,2R,3S)-3-Amino-4-cyclohexyl-1-cyclopropyl-1,2-butandiol das (RS)-N-[(1S,2R,3S)-1-(Cyclohexylmethyl)-3-cyclopropyl -2,3-dihydroxypropyl]-8-isopropyl-6-(3-pyridyl) -α-(3-pyridylmethyl)-s-triazolo[4,3-a]pyrazin-3-acetamid als 1:1-Epimerengemisch, MS: 598 $(M+H)^+$;
- aus rac-8-Isopropyl-α-(imidazol-1-ylmethyl) -6-(3-pyridyl)-s-triazolo[4,3-a]pyrazin-3-essigsäure und (1S,2R,3S)-3-Amino-4-cyclohexyl-1-cyclopropyl-1,2-butandiol die beiden Epimeren (S oder R)-N-[-(1S,2R,3S)-1-(Cyclohexylmethyl) -3-cyclopropyl-2,3-dihydroxypropyl]-8-isopropyl -α-(imidazol-4-ylmethyl)-6-(3-pyridyl) -s-triazolo[4,3-a]pyrazin-3-acetamid und (R oder S)-N-[(1S,2R,3S)-1-(Cyclohexylmethyl) -3-cyclopropyl-2,3-dihydroxypropyl]-8-isopropyl -α-(imidazol-4-ylmethyl)-6-(3-pyridyl) -s-triazolo[4,3-a]pyrazin-3-acetamid als jeweils hellgelbe Festkörper, MS: 587 $(M+H)^+$;
- aus rac-8-Isobutyl-6-(3-pyridyl)-α-(3-pyridylmethyl) -s-triazolo[4,3-a]pyrazin-3-essigsäure und (1S,2R,3S)-3-Amino-4-cyclohexyl-1-cyclopropyl-1,2-butandiol das 3:1-Epimerengemisch (RS)-N-[-(1S,2R,3S)-1-(Cyclohexylmethyl)-3-cyclopropyl -2,3-dihydroxypropyl]-8-isobutyl-α-(3-pyridylmethyl) -6-(3-pyridyl)-s-triazolo[4,3-a]pyrazin-3-acetamid und das weniger polare, reine Epimere (S oder R)-N-[-(1S,2R,3S)-1-(Cyclohexylmethyl) -3-cyclopropyl-2,3-dihydroxypropyl]-8-iobutyl -α-(3-pyridylmethyl)-6-(3-pyridyl) -s-triazolo[4,3-a]pyrazin-3-acetamid als jeweils hellgelbe Festkörper, MS: 612 $(M+H)^+$;
- aus rac-8-Isobutyl-α-(imidazol-1-ylmethyl)-6-(3-pyridyl) -s-triazolo[4,3-a]pyrazin-3-essigsäure und (1S,2R,3S)-3-Amino-4-cyclohexyl-1-cyclopropyl-1,2-butandiol die beiden Epimeren (S oder R)-N-[-(1S,2R,3S)-1-(Cyclohexylmethyl) -3-cyclohexyl-2,3-dihydroxypropyl] -α-(imidazol-4-ylmethyl)-8-isobutyl-6-(3-pyridyl) -s-triazolo[4,3-a]pyrazin-3-acetat und (R oder S)-N-[(1S,2R,3S)-1-(Cyclohexylmethyl) -3-cyclohexyl-2,3-dihydroxypropyl] -α-(imidazol-4-ylmethyl)-8-isobutyl-6-(3-pyridyl) -s-triazolo[4,3-a]pyrazin-3-acetat jeweils als Festkörper, MS: 601 $(M+H)^+$;
- aus 8-Isobutyl-6-(3-pyridyl)-s-triazolo[4,3-a]pyrazin-3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl -2,3-dihydroxypropyl]-8-isobutyl-6-(3-pyridyl) -s-triazolo[4,3-a]pyrazin-3-acetamid als Festkörper, MS: 521 $(M+H)^+$;
- aus rac-6-(4-Pyridyl)-α-(3-pyridylmethyl)-8-propyl -s-triazolo[4,3-a]pyrazin-3-essigsäure und (1S,2R,3S)-3-Amino-4-cyclohexyl-1-cyclopropyl-1,2-butandiol das 1:1-Epimerengemisch (RS)-N-[-(1S,2R,3S)-1-(Cyclohexylmethyl)-3-cyclopropyl -2,3-dihydroxypropyl]-8-propyl-6-(4-pyridyl) -α-(3-pyridylmethyl)-s-triazolo[4,3-a]pyrazin-3-acetamid als Festkörper, MS: 598 $(M+H)^+$;
- aus rac-α-(Imidazol-4-ylmethyl)-8-propyl-6-(4-pyridyl) -s-triazolo[4,3-a]pyrazin-3-essigsäure und (1S,2R,3S)-3-Amino-4-cyclohexyl-1-cyclopropyl-1,2-butandiol das (RS)-N-[(1S,2R,3S)-1-(Cyclohexylmethyl)-3-cyclopropyl -2,3-dihydroxypropyl]-α-(imidazol-4-ylmethyl) -8-propyl-6-(4-pyridyl)-s-triazolo[4,3-a]pyrazin-3-acetamid als 1:1-Epimerengemisch, MS: 587 $(M+H)^+$;
- aus 8-Propyl-6-(4-pyridyl)-s-triazolo[4,3-a]pyrazin -3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl -2,3-dihydroxypropyl]-8-propyl-6-(4-pyridyl) -s-triazolo[4,3-a]pyrazin-3-carboxamid als hellgelber Festkörper, MS: 507 $(M+H)^+$;
- aus 6-Phenyl-8-propyl-s-triazolo[4,3-b]pyridazin-3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl -2,3-dihydroxypropyl]-6-phenyl-8-propyl -s-triazolo[4,3-b]pyridazin-3-acetamid als Festkörper, MS: 508 $(M+H)^+$;
- aus rac-6-Phenyl-8-propyl-α-(3-pyridylmethyl) -s-triazolo[4,3-b]pyridazin-3-essigsäure und (1S,2R,3S)-3-Amino-4-cyclohexyl-1-cyclopropyl-1,2-butandiol das (RS)-N-[(1S,2R,3S)-1-(Cyclohexylmthyl)-3-cyclopropyl -2,3-dihydroxypropyl]-6-phenyl-8-propyl -α-(3-pyridylmethyl)-s-triazolo[4,3-b]pyridazin-3-carboxamid als Festkörper, MS: 598 $(M+H)^+$;
- aus 8-Benzyl-6-phenyl-s-triazolo[4,3-b]pyridazin-3-essigsäure und (1S,2R,3S)-3-Amino-4-cyclohexyl-1-cyclopropyl-1,2-butandiol das 8-Benzyl-N-[(1S,2R,3S)-1-(cyclohexylmethyl) -3-cyclopropyl-2,3-dihydroxypropyl]-6-phenyl -s-triazolo[4,3-b]pyridazin-3-acetamid, MS: 482 $(M-C_4H_7O)^+$;
- aus (RS)-8-Benzyl-6-phenyl-α-(3-pyridylmethyl) -s-triazolo[4,3-b]pyridazin-3-essigsäure und

(1S,2R,3S)-3-Amino-4-cyclohexyl-1-cyclopropyl-1,2-butandiol das (RS)-8-Benzyl-N-[(1S,2R,3S)-1-(cyclohexylmethyl) -3-cyclopropyl-2,3-dihydroxypropyl]-6-phenyl -α-(3-pyridylmethyl)-s-triazolo[4,3-b]-pyridazin-3-acetamid, MS: 645 $(M+H)^+$;

- aus rac-8-Propyl-6-(3-pyridyl)-α-(3-pyridylmethyl) -s-triazolo[4,3-a]pyrazin-3-essigsäure und (2S,3R,4S)-2-Amino-1-cyclohexyl-6-methyl-3,4-heptandiol (J.Med.Chem. 31 (12), 2277, 1988) die beiden Epimeren (S oder R)-N-[(1S,2R,3S)-1-(Cyclohexylmethyl)-2,3-dihydroxy -5-methylhexyl]-8-propyl-6-(3-pyridyl) -α-(3-pyridylmethyl)-s-triazolo[4,3-a]pyrazin-3-acetamid und (R oder S)-N-[(1S,2R,3S)-1-(Cyclohexylmethyl)-2,3-dihydroxy -5-methylhexyl]-8-propyl-6-(3-pyridyl) -α-(3-pyridylmethyl)-s-triazolo-[4,3-a]pyrazin-3-acetamid, MS: jeweils 614 $(M+H)^+$;
- aus rac-8-Propyl-6-(3-pyridyl)-α-(3-pyridylmethyl) -s-triazolo[4,3-a]pyrazin-3-essigsäure und (1S,2R,3S)-3-Amino-1-cyclopropyl-4-phenyl-1,2-butandiol das 1:1-Epimerengemisch (RS)-N-[-(1S,2R,3S)-1-(Cyclohexylmethyl)-3-cyclopropyl -2,3-dihydroxypropyl]-8-propyl-α-(3-pyridylmethyl) -6-(3-pyridyl)-s-triazolo[4,3-a]pyrazin-3-acetamid, MS: 592 $(M+H)^+$;
- aus rac-8-Benzyl-6-(3-pyridyl)-α-(3-pyridylmethyl) -s-triazolo[4,3-a]pyrazin-3-essigsäure und (1S,2R,3S)-3-Amino-4-cyclohexyl-1-cyclopropyl-1,2-butandiol das 1:1-Epimerengemisch (RS)-8-Benzyl-N-[(1S,2R,3S)-1-(cyclohexylmethyl) -3-cyclopropyl-2,3-dihydroxypropyl]-6-(3-pyridyl) -α-(3-pyridylmethyl)-s-triazolo[4,3-a]pyrazin-3-acetamid, MS: 646 $(M+H)^+$;
- aus rac-8-Benzyl-α-(imidazol-4-ylmethyl)-6-(3-pyridyl) -s-triazolo[4,3-a]pyrazin-3-essigsäure und (1S,2R,3S)-3-Amino-4-cyclohexyl-1-cyclopropyl-1,2-butandiol das 1:1-Epimerengemisch (RS)-8-Benzyl-N-[(1S,2R,3S)-1-(cyclohexylmethyl) -3-cyclopropyl-2,3-dihydroxypropyl]-α-(imidazol -4-ylmethyl)-6-(3-pyridyl)-s-triazolo[4,3-a]pyrazin -3-acetamid, MS: 635 $(M+H)^+$;
- aus 8-Benzyl-6-(3-pyridyl)-s-triazolo[4,3-a]pyrazin-3-essigsäure und (1S,2R,3S)-3-Amino-4-cyclohexyl-1-cyclopropyl-1,2-butandiol das 8-Benzyl-N-[ (1S,2R,3S)-1-(cyclohexylmethyl)-3-cyclopropyl -2,3-dihydroxypropyl]-6-(3-pyridyl) -s-triazolo[4,3-a]pyrazin-3-acetamid als Festkörper, MS: 555 $(M+H)^+$;
- aus rac-8-Propyl-6-(3-pyridyl)-α-(3-pyridylmethyl) -s-triazolo[4,3-a]pyrazin-3-essigsäure und (2S,3R,4S)-2-Amino-1-cyclohexyl-5-methyl-3,4-hexandiol (EP-A 0.332.008) die beiden Epimeren (R oder S)-N-[(1S,2R,3S)-1-(Cyclohexylmethyl) -2,3-dihydroxy-4-methylpentyl]-8-oxo-propyl -6-(3-pyridyl)-α-(3-pyridylmethyl) -s-triazolo[4,3-a]pyrazin-3-acetamid und (S oder R)-N-[(1S,2R,3S)-1-(Cyclohexylmethyl) -2,3-dihydroxy-4-methylpentyl]-8-oxo-propyl -6-(3-pyridyl)-α-(3-pyridylmethyl) -s-triazolo[4,3-a]pyrazin-3-acetamid, MS: jeweils 600 $(M+H)^+$;
- aus rac-α-(Imidazol-4-ylmethyl)-8-propyl-6-(3-pyridyl) -s-triazolo[4,3-a]pyrazin-3-essigsäure und (1S,2R,3S)-3-Amino-1-cyclopropyl-4-phenyl-1,2-butandiol das 2:1-Epimerengemisch (RS)-N-[-(1S,2R,3S)-1-(Cyclohexylmethyl)-3-cyclopropyl -2,3-dihydroxypropyl]-α-(imidazol-4-ylmethyl) -8-propyl-6-(3-pyridyl)-s-triazolo[4,3-a]pyrazin-3-acetamid als Festkörper, MS: 580 $(M)^+$;
- aus rac-α-(Imidazol-4-ylmethyl)-8-propyl-6-(3-pyridyl) -s-triazolo[4,3-a]pyrazin-3-essigsäure und (2S,3R,4S)-2-Amino-1-cyclohexyl-6-methyl-3,4-heptandiol die beiden Epimeren (R oder S)-N-[-(1S,2R,3S)-1-(Cyclohexylmethyl) -2,3-dihydroxy-4-methylhexyl]-α-(imidazol -4-ylmethyl)-8-propyl-6-(3-pyridyl) -s-triazolo[4,3-a]pyrazin-3-acetamid und (S oder R)-N-[(1S,2R,3S)-1-(Cyclohexylmethyl) -2,3-dihydroxy-4-methylhexyl]-α-(imidazol -4-ylmethyl)-8-propyl-6-(3-pyridyl) -s-triazolo[4,3-a]pyrazin-3-acetamid, MS: jeweils 603 $(M+H)^+$;
- aus rac-α-(Imidazol-4-ylmethyl)-8-propyl-6-(3-pyridyl) -s-triazolo[4,3-a]pyrazin-3-essigsäure und (2S,3R,4S)-2-Amino-1-cyclohexyl-5-methyl-3,4-hexandiol die beiden Epimeren (R oder S)-N-[-(1S,2R,3S)-1-(cyclohexylmethyl) -2,3-dihydroxy-4-methylpentyl]-α-(imidazol -4-ylmethyl)-8-propyl-6-(3-pyridyl) -s-triazolo[4,3-a]pyrazin-3-acetamid und (S oder R)-N-[(1S,2R,3S)-1-(cyclohexylmethyl)-2,3-dihydroxy -4-methylpentyl]-α-(imidazol-4-ylmethyl)-8-propyl -6-(3-pyridyl)-s-triazolo[4,3-a]pyrazin-3-acetamid, MS: jeweils 589 $(M+H)^+$;
- aus rac-8-Propyl-α-(3-pyridylmethyl)-6-(3-pyridyl) -s-triazolo[4,3-b]pyridazin-3-essigsäure und (1S,2R,3S)-3-Amino-4-cyclohexyl-1-cyclopropyl-1,2-butandiol das (RS)-N-[(1S,2R,3S)-1-(Cyclohexylmethyl)-3-cyclopropyl -2,3-dihydroxypropyl]-8-propyl-α-(3-pyridylmethyl) -6-(3-pyridyl)-s-triazolo[4,3-b]pyridazin-3-acetamid als 1:1-Epimerengemisch, MS: 598 $(M+H)^+$;
- aus rac-8-Propyl-α-(3-pyridylmethyl)-6-(2-thienyl) -s-triazolo[4,3-b]pyridazin-3-essigsäure und (1S,2R,3S)-3-Amino-4-cyclohexyl-1-cyclopropyl-1,2-butandiol die beiden Epimeren (S oder R)-N-[-(1S,2R,3S)-1-(Cyclohexylmethyl)-3-cyclopropyl -2,3-dihydroxypropyl]-8-propyl-α-(3-pyridylmethyl) -6-(2-thienyl)-s-triazolo[4,3-b]pyridazin-3-acetamid und (R oder S)-N-[(1S,2R,3S)-1-(Cyclohexylmethyl)-3-cyclopropyl -2,3-dihydroxypropyl]-8-propyl-α-(3-pyridylmethyl) -6-(2-thienyl)-s-triazolo[4,3-b]pyridazin-3-acetamid jeweils als Festkörper, MS: 603 $(M+H)^+$;
- aus 8-Propyl-6-(2-thienyl)-s-triazolo[4,3-b]pyridazin-3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl -2,3-dihydroxypropyl]-8-propyl-6-(2-thienyl) -s-triazolo[4,3-b]pyridazin-3-acetamid als Festkörper, MS: 511 $(M)^+$;

- aus rac-8-Propyl-6-(3-pyridyl)-α-(3-pyridylmethyl) -s-triazolo[4,3-a]pyrazin-3-essigsäure und (1R oder S,2R oder S)-1-[(1R,2S)-1-Amino-3-cyclohexyl-1-hydroxypropyl] -1,2-cyclohexandiol die beiden Epimeren (R oder S)-N-[(1S,2R)-1-(Cyclohexylmethyl)-2-hydroxy-2-[(1R oder S,2R oder S)-1,2-dihydroxyphenyl]äthyl]-8-propyl -α-(3-pyridylmethyl)-8-propyl-s-triazolo[4,3-a]pyrazin -3-acetamid und (S oder R)-N-[(1S,2R)-1-(Cyclohexylmethyl)-2-hydroxy-2-[(1R oder S,2R oder S)-1,2-dihydroxyphenyl]-äthyl]-8-propyl -α-(3-pyridylmethyl)-8-propyl-s-triazolo[4,3-a]pyrazin -3-acetamid jeweils als Festkörper, MS: 642 $(M+H)^+$;
- aus (RS)-8-[(RS)-1-Methylpropyl]-α-(3-pyridylmethyl) -6-(3-pyridyl)-s-triazolo[4,3-a]pyrazin-3-essigsäure und (1S,2R,3S)-3-Amino-4-cyclohexyl-1-cyclopropyl-1,2-butandiol die beiden Epimerengemische (S oder R)-N-[(1S,2R,3S)-1-(Cyclohexylmethyl)-3-cyclopropyl -2,3-dihydroxypropyl]-8-[(RS)-1-methylpropyl] -α-(3-pyridylmethyl)-6-(3-pyridyl) -s-triazolo[4,3-a]pyrazin-3-acetamid und (RS)-N-[(1S,2R,3S)-1-(Cyclohexylmethyl)-3-cyclopropyl -2,3-dihydroxypropyl]-8-[(RS)-1-methylpropyl] -α-(3-pyridylmethyl)-6-(3-pyridyl) -s-triazolo[4,3-a]pyrazin-3-acetamid als jeweils hellgelbe Festkörper, MS: 612 $(M+H)^+$;
- aus rac-8-(1-Methylpropyl)-6-(3-pyridyl) -s-triazolo[4,3-a]pyrazin-3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl -2,3-dihydroxypropyl]-8-[(RS)-1-methylpropyl] -6-(3-pyridyl)-s-triazolo[4,3-a]pyrazin-3-carboxamid als Festkörper, MS: 520, $(M)^+$;
- aus rac-α-(Imidazol-4-ylmethyl)-8-[(RS)-1-methylpropyl] -6-(3-pyridyl)-s-triazolo[4,3-a]pyrazin-3-essigsäure und (1S,2R,3S)-3-Amino-4-cyclohexyl-1-cyclopropyl-1,2-butandiol die beiden 1:1-Epimerengemische (S oder R)-8-[(RS)-1-Methylpropyl]-N-[(1S,2R,3S)-1-(cyclohexylmethyl) -3-cyclopropyl-2,3-dihydroxypropyl]-α-(imidazol -4-ylmethyl)-6-(3-pyridyl)-s-triazolo[4,3-a]pyrazin-3-acetamid und (R oder S)-8-[(RS)-1-Methylpropyl]-N-[(1S,2R,3S)-1-(cyclohexylmethyl) -3-cyclopropyl-2,3-dihydroxypropyl]-α-(imidazol -4-ylmethyl)-6-(3-pyridyl)-s-triazolo[4,3-a]pyrazin -3-acetamid als jeweils hellgelbe Festkörper, MS: 601 $(M+H)^+$;
- aus rac-α-Methyl-8-propyl-6-(3-pyridyl)-s-triazolo[4,3-a]pyrazin-3-essigsäure und (1S,2R,3S)-3-Amino-4-cyclohexyl-1-cyclopropyl-1,2-butandiol das (RS)-N-[(1S,2R,3S)-1-(Cyclohexylmethyl)-3-cyclopropyl-2,3-dihydroxypropyl]-α-methyl-8-propyl-6-(3-pyridyl)-s-triazolo[4,3-a]pyrazin-3-acetamid als 1:1 Epimerengemisch, MS: 521 $(M+H)^+$ und
- aus rac-α-Methyl-8-[(RS)-1-methylpropyl]-6-(3-pyridyl)-s-triazolo[4,3-a]pyrazin-3-essigsäure und (1S,2R,3S)-3-Amino-4-cyclohexyl-1-cyclopropyl-1,2-butandiol das (RS)-8-[(RS)-1-Methylpropyl]-N-[-(1S,2R,3S)-1-(cyclohexylmethyl)-3-cyclopropyl-2,3-dihydroxypropyl]-α-methyl-6-(3-pyridyl)-s-triazolo-[4,3-a]pyrazin-3-acetamid als Diastereomerengemisch, MS: 535 $(M+H)^+$.

Die als Ausgangsstoffe eingesetzten Säuren und Amindiole wurden wie folgt hergestellt:

rac-α-(Imidazol-4-ylmethyl)-8-propyl-6-(3-pyridyl) -s-triazolo[4,3-a]pyrazin-3-essigsäure

In analoger Weise wie in Beispiel 1 beschrieben erhält man durch Kondensation von 2-Hydrazino-3-propyl-5-(3-pyridyl)pyrazin mit Diäthyl (imidazol-4-yl-methyl)malonat (J.Chem.Soc. 99, (1911) 1390) das Aethyl rac-α-(imidazol-4-ylmethyl)-8-propyl-6-(3-pyridyl) -s-triazolo[4,3-a]pyrazin-3-acetat, MS: 405 $(M)^+$, welches durch Verseifung, analog zu Beispiel 1, in die obige Säure übergeführt wird.

8-Propyl-6-(3-pyridyl)-s-triazolo[4,3-a]pyrazin-3-essigsäure

In analoger Weise wie in Beispiel 1 beschrieben erhält man durch Kondensation von 2-Hydrazino-3-propyl-5-(3-pyridyl)pyrazin mit Malonsäurediäthylester in Toluol anstatt Xylol das Aethyl 8-propyl-6-(3-pyridyl)-s-triazolo[4,3-a]pyrazin-3-acetat, MS: 325 $(M)^+$, und durch entsprechende basische Verseifung die gewünschte Säure, MS: 269 $(M-C_2H_4)^+$.

rac-8-Isopropyl-6-(3-pyridyl)-α-(3-pyridylmethyl) -s-triazolo[4,3-a]pyrazin-3-essigsäure

In analoger Weise zu Beispiel 1 erhält man durch Kondensation von 3-(2-Amino-1,1-diäthoxyäthyl)-pyridin mit 3-Methyl-2-oxobutansäure das N-[2,2-Diäthoxy-2-(3-pyridyl)äthyl]-3,3-dimethyl -2-oxobutyramid. Saure Hydrolyse zu 3-Methyl-N-(nicotinoylmethyl)-2-oxobutyramid und anschliessender Ringschluss ergibt 3-Isopropyl-5-(3-pyridyl)-2(1H)-pyrazinon als gelber, kristalliner Festkörper, Schmelzpunkt 200-202° (aus Essigester). Die Ueberführung in das entsprechende Chlorid 2-Chlor-3-isopropyl-5-(3-pyridyl)pyrazin erfolgt

analog zu Beispiel 1.

Hydrazinolyse liefert 2-Hydrazino-3-isopropyl-5-(3-pyridyl)pyrazin als beige Kristalle, Schmelzpunkt 164-165° (aus Aethanol/Wasser) und anschliessende Kondensation mit Diäthyl (3-pyridyl)malonat das Aethyl rac-8-isopropyl-6-(3-pyridyl)-α-(3-pyridylmethyl) -s-triazolo[4,3-a]pyrazin-3-acetat als hellgelben Festkörper, MS: 416 $(M)^+$, welches durch basische Verseifung in obige Säure übergeführt wird.

rac-8-Isopropyl-α-(imidazol-4-ylmethyl)-6-(3-pyridyl) -s-triazolo[4,3-a]pyrazin-3-essigsäure

In analoger Weise zu Beispiel 1 erhält man durch Kondensation von 2-Hydrazino-3-isopropyl-5-(3-pyridyl)pyrazin mit Diäthyl (imidazol-4-ylmethyl)malonat das Aethyl rac-8-isopropyl-α-(imidazol-4-ylmethyl)-6-(3-pyridyl) -s-triazolo[4,3-a]pyrazin-3-acetat, MS: 405 $(M)^+$, welches durch alkalische Verseifung in obige Säure übergeführt wird.

rac-8-Isobutyl-α-(3-pyridylmethyl)-6-(3-pyridyl) -s-triazolo[4,3-a]pyrazin-3-essigsäure

In analoger Weise zu Beispiel 1 erhält man durch Kondensation von 3-(2-Amino-1,1-diäthoxyäthyl)-pyridin mit 4-Methyl-2-oxopentansäure das N-(2,2-Diäthoxy-2-(3-pyridyl)äthyl]-4-methyl-2-oxovaleramid als farblosen, amorphen Festkörper. Saure Hydrolyse liefert 4-Methyl-N-(nicotinoylmethyl)-2-oxovaleramid und anschliessender Ringschluss 3-Isobutyl-5-(3-pyridyl)-2(1H)-pyrazinon als kristallinen Festkörper, Schmelzpunkt 195-196° (aus Essigester). Die Ueberführung in das entsprechende Chlorid 2-Chlor-3-isobutyl-5-(3-pyridyl)pyrazin erfolgt ebenfalls analog zu Beispiel 1.

Hydrazinolyse des Chlorids liefert 2-Hydrazino-3-isobutyl-5-(3-pyridyl)pyrazin als beige Kristalle, Schmelzpunkt 154-155° (aus Aethanol/Wasser) und anschliessende Kondensation mit Diäthyl (3-pyridyl)-malonat das Aethyl rac-8-isobutyl-α-(3-pyridylmethyl) -6-(3-pyridyl)-s-triazolo[4,3-a]pyrazin-3-acetat, MS: 430 $(M)^+$, welches durch basische Verseifung in obige Säure übergeführt wird.

rac-α-(Imidazol-4-ylmethyl)-8-isobutyl-6-(3-pyridyl) -s-triazolo[4,3-a]pyrazin-3-essigsäure

In analoger Weise zu Beispiel 1 erhält man durch Kondensation von 2-Hydrazino-3-isobutyl-5-(3-pyridyl)pyrazin mit Diäthyl (imidazol-4-ylmethyl)malonat das Aethyl rac-α-(imidazol-4-ylmethyl)-8-isobutyl -6-(3-pyridyl)-s-triazolo[4,3-a]pyrazin-3-acetat, MS: 419 $(M)^+$, das durch alkalische Verseifung in obige Säure übergeführt wird.

8-Isobutyl-6-(3-pyridyl)-s-triazolo[4,3-a]pyrazin-3-essigsäure

In analoger Weise zu Beispiel 1 erhält man durch Kondensation von 2-Hydrazino-3-isobutyl-5-(3-pyridyl)pyrazin mit Diäthylmalonat das Aethyl 8-isobutyl-6-(3-pyridyl)-s-triazolo[4,3-a]pyrazin-3-acetat, MS: 339 $(M)^+$, das durch Verseifung in obige Säure übergeführt wird: MS: 267 $(M-CO_2)^+$.

rac-8-Benzyl-6-(3-pyridyl)-α-(3-pyridylmethyl) -s-triazolo[4,3-a]pyrazin-3-essigsäure

In analoger Weise zu Beispiel 1 erhält man durch Kondensation von 3-(2-Amino-1,1-diäthoxyäthyl)-pyridin mit Phenylbrenztraubensäure das N-[2,2-Diäthoxy-2-(3-pyridyl)äthyl]-3-phenylpyruvamid als amorphen, gelben Festkörper. Anschliessende saure Hydrolyse zu 3-Phenyl-N-[[(3-pyridyl)carbonyl]methyl]-pyruvamid und Ringschluss ergibt das 3-Benzyl-5-(3-pyridyl)-2(1H)-pyrazinon als Festkörper, Schmelzpunkt 188-190° (aus Essigester).

Chlorierung zu 2-Benzyl-3-chlor-6-(3-pyridyl)pyrazin und Hydrazinolyse liefert das 2-Benzyl-3-hydrazino-6-(3-pyridyl)pyrazin als beigen, kristallinen Festkörper. Schmelzpunkt 183-184° (aus Methanol/Wasser).

Kondensation mit Diäthyl (3-pyridyl)malonat ergibt schliesslich das Aethyl rac-8-benzyl-α-(3-pyridylmethyl)-6-(3-pyridyl) -s-triazolo[4,3-a]pyrazin-3-acetat, MS: 464 $(M)^+$, das durch basische Verseifung in obige Säure übergeführt wird.

rac-8-Benzyl-α-(imidazol-4-ylmethyl)-6-(3-pyridyl) -s-triazolo[4,3-a]pyrazin-3-essigsäure

In analoger Weise wie in Beispiel 1 beschrieben erhält man durch Kondensation von 2-Benzyl-3-hydrazino-6-(3-pyridyl)pyrazin mit Diäthyl (imidazol-4-ylmethyl)malonat das Aethyl rac-8-benzyl-α-(imidazol-4-ylmethyl)-6-(3-pyridyl) -s-triazolo[4,3-a]pyrazin-3-acetat, MS: 453 $(M)^+$, welches durch alkalische Verseifung in obige Säure übergeführt wird.

8-Benzyl-6-(3-pyridyl)-s-triazolo[4,3-a]pyrazin-3-essigsäure

In analoger Weise zu Beispiel 1 erhält man durch Kondensation von 2-Benzyl-3-hydrazino-6-(3-pyridyl)-pyrazin mit Diäthylmalonat das Aethyl 8-benzyl-6-(3-pyridyl)-s-triazolo[4,3-a]pyrazin-3-acetat, welches durch basische Verseifung in obige Säure übergeführt wird, MS: 301 $(M-CO_2)^+$.

rac-6-(4-Pyridyl)-a-(3-pyridyl)-8-propyl -s-triazolo[4,3-a]pyrazin-3-essigsäure

In analoger Weise zu Beispiel 1 erhält man durch Kondensation von 4-(2-Amino-1,1-diäthoxyäthyl)-pyrimidin [Org. Synthesis 64, 19 (1986)] mit 2-Oxopentansäure das N-[2,2-Diäthoxy-2-(4-pyridyl)äthyl]-2-oxovaleramid als weissen Festkörper, Schmelzpunkt 95-96°. Anschliessende saure Hydrolyse zu N-(Isonicotinoylmethyl)-2-oxovaleramid und Ringschluss ergibt das 3-Propyl-5-(4-pyridyl)-2-pyrazinon als gelbe Kristalle, Schmelzpunkt 237-238° (Zers.).

Chlorierung zu 2-Chloro-3-propyl-5-(4-pyridyl)pyrazin und Hydrazinolyse liefert das 2-Hydrazino-3-propyl-5-(4-pyridyl)pyrazinals roten, kristallinen Festkörper, Schmelzpunkt 216-217° (aus Aethanol/Wasser).

Kondensation mit Diäthyl (3-pyridyl)malonat, analog zu Beispiel 1, ergibt schliesslich das Aethyl rac-6-(4-Pyridyl)-α-(3-pyridyl)-8-propyl-s-triazolo[4,3-a]pyrazin-3-acetat, MS: 416 $(M)^+$, das durch basische Verseifung in obige Säure übergeführt wird.

rac-α-(Imidazol-4-ylmethyl)-8-propyl-6-(4-pyridyl) -s-triazolo[4,3-a]pyrazin-3-essigsäure

Analog zu Beispiel 1 erhält man durch Kondensation von 2-Hydrazino-3-propyl-5-(4-pyridyl)pyrazin mit Diäthyl (imidazol-4-ylmethyl)malonat das Aethyl rac-α-(imidazol-4-ylmethyl)-8-propyl-6-(4-pyridyl) -s-triazolo-[4,3-a]pyrazin-3-acetat, MS: 405 $(M)^+$, welches durch basische Verseifung in obige Säure übergeführt wird.

8-Propyl-6-(4-pyridyl)-s-triazolo[4,3-a]pyrazin-3-essigsäure

Analog zu Beispiel 1 erhält man durch Kondensation von 2-Hydrazino-3-propyl-5-(4-pyridyl)pyrazin mit Diäthylmalonat das Aethyl 8-propyl-6-(4-pyridyl)-s-triazolo[4,3-a]pyrazin-3-acetat, MS: 325 $(M)^+$, welches durch basische Verseifung in obige Säure übergeführt wird.

6-Phenyl-8-propyl-s-triazolo[4,3-b]pyridazin-3-essigsäure

In analoger Weise zu Beispiel 1 erhält man durch Chlorierung von 6-Phenyl-4-propyl-3(2H)-pyridazinon [Chim.Ther. 6, 109 (1971)] das 3-Chlor-6-phenyl-4-propylpyridazin, MS: 232 $(M)^+$, welches durch Hydrazinolyse in das 3-Hydrazino-6-phenyl-4-propylpyridazin, MS: 228 $(M)^+$, übergeführt wird. Anschliessende Ringkondensation mit Diäthylmalonat zum Aethyl 6-phenyl-8-propyl-s-triazolo[4,3-b]pyridazin-3-acetat, MS: 324 $(M)^+$, gefolgt von basischer Verseifung ergibt obige Säure als Festkörper, MS: 252 $(M-CO_2)^+$.

rac-6-Phenyl-8-propyl-α-(3-pyridylmethyl)-s-triazolo[4,3-b]pyridazin-3-essigsäure

In analoger Weise zu Beispiel 1 erhält man durch Kondensation von 3-Hydrazino-6-phenyl-4-propylpyridazin mit Diäthyl (3-pyridyl)malonat das Aethyl rac-6-phenyl-8-propyl-α-(3-pyridylmethyl) -s-triazolo[4,3-b]-pyridazin-3-acetat, das durch basische Verseifung in obige Säure übergeführt wird, MS: 398 $(M+H)^+$.

8-Benzyl-6-phenyl-s-triazolo[4,3-b]pyridazin-3-essigsäure

In analoger Weise wie in Beispiel 1 beschrieben erhält man durch Ringkondensation von 4-Benzyl-3-hydrazino-6-phenylpyridazin [Indian J.Chem., 15B, 352 (1977)] mit Diäthylmalonat das Aethyl 8-benzyl-6-phenyl-s-triazolo[4,3-b]pyridazin-3-acetat, MS: 372 $(M)^+$, das basisch zu obiger Säure verseift wird, MS: 316 $(M-CO)^+$.

(RS)-8-Benzyl-6-phenyl-α-(3-pyridylmethyl)-s-triazolo[4,3-b]pyridazin-3-essigsäure

In analoger Weise erhält man durch Ringkondensation von 4-Benzyl-3-hydrazino-6-phenylpyridazin mit Diäthyl (3-pyridyl)malonat das Aethyl rac-8-benzyl-6-phenyl-α-(3-pyridylmethyl) -s-triazolo[4,3-b]pyridazin-3-acetat, das durch basische Verseifung in obige Säure übergeführt wird, MS: 436 $(M+H)^+$.

rac-8-(1-Methylpropyl)-6-(3-pyridyl)-s-triazolo[4,3-a]pyrazin-3-essigsäure

In analoger Weise zu Beispiel 1 erhält man durch Kondensation von 3-(2-Amino-1,1-diäthoxyäthyl)-pyridin mit 3-Methyl-2-oxopentansäure das rac-N-[2,2-Diäthoxy-2-(3-pyridyl)äthyl]-3-methyl -2-oxovaleramid als amorphen, farblosen Festkörper. Anschliessende saure Hydrolyse zu rac-3-Methyl-2-oxo-N-[(3-pyridyl-carbonyl)methyl]valeramid-hydrochlorid (beiger, kristalliner Festkörper, Schmelzpunkt 158-159° ) und Ringschluss ergibt das rac-3-(1-Methylpropyl)-5-(3-pyridyl)-2(1H)-pyrazinon als gelben Festkörper. Schmelzpunkt 146-148° (aus Acetonitril). Die Ueberführung in das rac-2-Chlor-3-(1-methylpropyl)-5-(3-pyridyl)pyrazin erfolgt ebenfalls analog zu Beispiel 1. Hydrazinolyse zu rac-2-Hydrazino-3-(1-methylpropyl)-5-(3-pyridyl)-pyrazin in Form beiger Kristalle, Schmelzpunkt 166-167° (aus Aethanol/Wasser) und anschliessende Kondensation mit Malonsäurediäthylester ergibt das Aethyl rac-8-(1-methylpropyl)-6-(3-pyridyl) -s-triazolo-[4,3-a]pyrazin-3-acetat, MS: 335 $(M)^+$, das durch Verseifung in obige Säure übergeführt wird, MS: 267 $(M-CO_2)^+$.

(RS)-8-[(RS)-1-Methylpropyl]-α-(3-pyridylmethyl)-6-(3-pyridyl)-s-triazolo[4,3-a]pyrazin-3-essigsäure

In analoger Weise zu Beispiel 1 erhält man durch Kondensation von rac-2-Hydrazino-3-(1-methylpropyl)-5-(3-pyridyl)pyrazin mit Diäthyl (3-pyridyl)malonat das Aethyl (RS)-8-[(RS)-1-methylpropyl]-α-(3-pyridylmethyl) -6-(3-pyridyl)-s-triazolo[4,3-a]pyrazin-3-acetat, MS: 430 $(M)^+$, das durch alkalische Verseifung in obige Säure übergeführt wird.

rac-α-(Imidazol-4-ylmethyl)-8-[(RS)-1-methylpropyl] -6-(3-pyridyl)-s-triazolo[4,3-a]pyrazin-3-essigsäure

In analoger Weise zu Beispiel 1 erhält man durch Kondensation von rac-2-Hydrazino-3-(1-methylpropyl)-5-(3-pyridyl)pyrazin mit Diäthyl (imidazol-4-ylmethyl)malonat das Aethyl (RS)-8-[(RS)-1-methylpropyl]-α-(imidazol-4-ylmethyl) -6-(3-pyridyl)-s-triazolo[4,3-a]pyrazin-3-acetat, MS: 419 $(M)^+$, das durch alkalische Verseifung in obige Säure übergeführt wird.

8-Propyl-6-(2-thienyl)-s-triazolo[4,3-b]pyridazin-3-essigsäure

Zu einer Suspension von 11.6 g (100 mMol) 2-Oxo-n-valeriansäure und 12.6 g (100 mMol) 2-Acetylthiophen in 75 ml Aethanol werden 11.2 g (200 mMol) Kaliumhydroxid in 75 ml Wasser bei 0° getropft, und die Suspension wird 48 Stunden bei 0° gehalten. Nach Abdampfen des Aethanols wird mit 25%iger Salzsäure sauer gestellt und dreimal mit Essigsäureäthylester extrahiert. Die vereinigten Essigsäureäthylesterextrakte werden getrocknet, filtriert und eingedampft. Nach Chromatographie an Kieselgel mit einem 5:1-Gemisch von Methylenchlorid und Methanol als Eluierungsmittel und Kristallisation aus Aether erhält man 10.0 g (41%) rac-α-Hydroxy-α-propyl-γ-oxo-2-thiophenbuttersäure als farbloses Pulver, Schmelzpunkt 128-131° , MS: 197 $(M-COOH)^+$.

Eine Lösung von 10 g rac-α-Hydroxy-α-propyl-γ-oxo-2-thiophenbuttersäure und 25 ml Hydrazinhydrat in 250 ml Aethanol werden 3 Stunden zum Rückfluss erhitzt. Die Lösung wird auf ca. 40 ml eingedampft, und die ausgefallenen Kristalle abgenutscht. Man erhält so 5.1 g (56%) 4-Propyl-6-(2-thienyl)-3(2H)-pyridazinon als farbloses Pulver. Schmelzpunkt 171-174° , MS: 220 $(M)^+$.

Chlorierung zu 3-Chlor-4-propyl-6-(2-thienyl)pyridazin, MS: 238 $(M)^+$, und Hydrazinolyse liefert das 3-Hydrazino-4-propyl-6-(2-thienyl)pyridazin, Schmelzpunkt 129-132° , analog zu Beispiel 1.

Anschliesende Ringkondensation mit Malonsäurediäthylester liefert 8-Propyl-6-(2-thienyl)-s-triazolo[4,3-b]pyridazin -3-essigsäureäthylester, welcher durch basische Verseifung in die gewünschte 8-Propyl-6-(2-thienyl)-s-triazolo[4,3-b]pyridazin-3-essigsäure übergeführt wird, MS: 258 $(M-CO_2)^+$.

rac-8-Propyl-α-(3-pyridylmethyl)-6-(2-thienyl)-s-triazolo[4,3-b]pyridazin-3-essigsäure

In analoger Weise zu Beispiel 1 erhält man durch Kondensation des oben beschriebenen 3-Hydrazino-4-propyl-6-(2-thienyl)pyridazins mit Diäthyl (3-pyridyl)malonat den rac-8-Propyl-α-(3-pyridylmethyl)-6-(2-thienyl) -s-triazolo[4,3-b]pyridazin-3-essigsäureäthylester, der durch alkalische Verseifung in die obige Säure übergeführt wird, MS: 349 $(M-CO_2)^+$.

rac-8-Propyl-α-(3-pyridylmethyl)-6-(3-pyridyl)-s-triazolo[4,3-b]pyridazin-3-essigsäure

In analoger Weise wie oben beschrieben erhält man durch Aldolkondensation von 2-Oxo-n-valeriansäure

mit 3-Acetyl-pyridin die γ-Oxo-α-[(E)-propyliden]-3-pyridinbuttersäure, MS: 219 $(M+H)^+$. Ringschluss mit Hydrazin liefert das 4-Propyl-6-(3-pyridyl)-3(2H)-pyridazinon, MS: 215 $(M)^+$, welches in das entsprechende Chlorid 3-Chlor-4-propyl-6-(3-pyridyl)pyridazin, MS: 233 $(M)^+$, übergeführt wird. Hydrazinolyse zu 3-Hydrazino-4-propyl-6-(3-pyridyl)pyridazin, MS: 229 $(M)^+$, und Kondensation mit Diäthyl (3-pyridyl)malonat liefert rac-8-Propyl-α-(3-pyridylmethyl) -6-(3-pyridyl)-s-triazolo[4,3-b]pyridazin -3-essigsäureäthylester, welcher durch basische Verseifung in die obige Säure übergeführt wird, MS: 388 $(M)^+$.

rac-α-Methyl-8-propyl-6-(3-pyridyl)-s-triazolo[4,3-a]pyrazin-3-essigsäure

In analoger Weise wie in Beispiel 1 beschrieben erhält man durch Kondensation von 2-Hydrazino-3-propyl-5-(3-pyridyl)pyrazin mit Diäthylmethylmalonat das Aethyl rac-α-methyl-8-propyl-6-(3-pyridyl)-s-triazolo[4,3-a]pyrazin-3-acetat, MS: 339 $(M)^+$, welches durch Verseifung analog zu Beispiel 1 in die obige Säure übergeführt wird, die direkt in die nächste Stufe eingesetzt wird.

rac-α-Methyl-8-[(RS)-1-methylpropyl]-6-(3-pyridyl)-s-triazolo[4,3-a]pyrazin-3-essigsäure

In analoger Weise wie in Beispiel 1 beschrieben erhält man durch Kondensation von 2-Hydrazino-3-[-(RS)-1-methylpropyl]-5-(3-pyridyl)pyrazin mit Diäthylmethylmalonat das Aethyl (RS)-8-[(RS)-1-Methylpropyl]-α-methyl-6-(3-pyridyl)-s-triazolo[4,3-a]pyrazin-3-acetat, MS: 353 $(M)^+$, welches durch Verseifung analog zu Beispiel 1 in die obige Säure übergeführt wird, die direkt in die nächste Stufe eingesetzt wird.

(1S,2R,3S)-3-Amino-1-cyclopropyl-4-phenyl-1,2-butandiol

Zu 3.03 g (124.5 mMol) Magnesium in absolutem Aether (10 ml) tropft man unter leichtem Rückfluss 9.96 ml (124.5 mMol) Bromcyclopropan in Aether (100 ml) innert 30 Minuten und erhitzt anschliessend 2 1/2 Stunden zum Rückfluss. Anschliessend fügt man innerhalb von 45 Minuten 6.25 g (22.63 mMol) tert.-Butyl [1S,2R)-1-benzyl-2-cyano-2-hydroxyäthyl]carbamat (EP-A 0.266.950) unter Rückfluss tropfenweise zu und erhitzt noch weitere 2 1/2 Stunden zum Rückfluss. Schliesslich lässt man auf 10° abkühlen, tropft 10%-ige Zitronensäure (100 ml) zu und extrahiert zweimal mit je 250 ml Aether. Nach üblicher Aufarbeitung der organischen Phase reinigt man das Rohprodukt durch Flashchromatographie an Kieselgel mit einem 9:1-Gemisch von Toluol und Essigester als Eluierungsmittel. Auf diese Weise erhält man 2.83 g (39%) tert.-Butyl [(1S,2R)-1-benzyl-2-(cyclopropylcarbonyl) -2-hydroxyäthyl]carbamat, MS: 246 $(M-C_3H_9O)^+$.

2.83 g (8.77 mMol) tert.-Butyl [(1S,2R)-1-benzyl-2-(cyclopropylcarbonyl) -2-hydroxyäthyl)carbamat löst man in Methylenchlorid (130 ml), fügt Essigsäure hinzu (3 ml) und versetzt schliesslich portionsweise bei 0-10° mit 332 mg (8.78 mMol) Natriumborhydrid. Man rührt weitere 2 Stunden bei 5° und verteilt die Reaktionslösung schliesslich zwischen 2N Natriumbicarbonat-Lösung und Methylenchlorid. Nach üblicher Aufarbeitung der organischen Phase kristallisiert man den Rückstand aus Aether/Hexan, wobei man 2.1 g (74%) tert-Butyl [(1S,2R,3S)-1-(cyclohexylmethyl)-3-cyclopropyl -2,3-dihydroxypropyl]carbamat erhält, Schmelzpunkt 83-85°.

2.0 g (6.23 mMol) tert-Butyl [(1S,2R,3S)-1-(cyclohexylmethyl)-3-cyclopropyl -2,3-dihydroxypropyl]-carbamat löst man in Methanol (20 ml), fügt 2N Salzsäure (20 ml) zu und erhitzt die Lösung 90 Minuten auf 50°.

Man neutralisiert durch Zugabe von 1N Natronlauge (40 ml) und engt unter vermindertem Druck zur Trockene ein. Den Rückstand verteilt man zwischen Wasser und Methylenchlorid und arbeitet die organisch Phase wie üblich auf. Man erhält so das gewünschte (1S,2R,3S)-3-Amino-1-cyclopropyl-4-phenyl-1,2-butandiol als weissen, kristallinen Festkörper, Ausbeute 0.7 g (50.8%), MS: 150 $(M-C_4H_8O)^+$.

(1R oder S,2R oder S)-1-[(1R,2S)-1-Amino-3-cyclohexyl -1-hydroxypropyl]-1,2-cyclohexandiol

Man löst 1.28 g (5 mMol) tert-Butyl (1S)-(2-cyclohexyl-1-formyläthyl)carbamat (EP-A 0.332.008) in 25 ml Aether und tropft bei -78° 70 ml (7 Moläq.) einer 0.5 molaren 1-Lithium-1-cyclohexen-Lösung (J.C.S. 1950, 2014) in Aether zu. Danach lässt man 1 1/2 Stunden bei -78° reagieren und erhitzt anschliessend die Reaktionslösung weitere 48 Stunden zum Rückfluss. Nach üblicher wässriger Aufarbeitung erhält man das gewünschte Produkt als Epimerengemisch, das durch Chromatographie an Kieselgel in die einzelnen Komponenten aufgetrennt wird (Laufmittel: 9:1-Gemisch von Toluol und Essigester). Auf diese Weise erhält man 270 mg (16%) tert-Butyl [(1S,2S)-2-(1-cyclohexen-1-yl)-1-(cyclohexylmethyl)äthyl]carbamat als Kristalle und 800 mg (47%) tert-Butyl [(1S,2R)-2-(1-cyclohexen-1-yl)-1-(cyclohexylmethyl)äthyl]carbamat als Oel,

$R_F$:0.4 bzw. 0.35 (4:1-Gemisch von Toluol und Essigester als Laufmittel).

Man löst 260 mg tert-Butyl [(1S,2S)-2-(1-cyclohexen-1-yl)-1-(cyclohexylmethyl)äthyl]carbamat (bzw. des epimeren tert-Butyl [(1S,2R)-2-(1-cyclohexen-1-yl)-1-(cyclohexylmethyl)äthyl]carbamats) in 2,2-Dimethoxypropan (10 ml), fügt 15 mg p-Toluolsulfonsäuremonohydrat zu und rührt die Lösung 3 Stunden bei Raumtemperatur. Nach üblicher wässriger Aufarbeitung chromatographiert man den Rückstand an Kieselgel (Essigester/Toluol; 9:1) und erhält 270 mg (39%) tert-Butyl (4S,5S)-5-(1-cyclohexen-1-yl) -4-(cycloheylmethyl)-2,2-dimethyl-3-oxazolidincarboxylat als Oel, $R_F$:0.4 (9:1-Gemisch von Essigester und Toluol als Laufmittel).

340 mg (0.9 mMol) tert-Butyl (4S,5S)-5-(1-cyclohexen-1-yl)-4-(cycloheylmethyl) -2,2-dimethyl-3-oxazolidincarboxylat in 36 ml eines 3:1-Gemisches von Aeton und Wasser versetzt man mit 2.7 mMol 4-Methylmorpholin-4-oxid-monohydrat und anschliessend mit 3.4 ml Osmiumtetroxid-Lösung (1 g Osmiumtetroxid in 199 ml t-Butanol und 1 ml t-Butylhydroperoxid, 70% in Wasser) und rührt die Lösung 4 Stunden bei Raumtemperatur. Anschliessend versetzt man mit 3.4 ml 38%-iger Natriumbisulfit-Lösung, engt unter vermindertem Druck ein, extrahiert mit Essigester und erhält nach üblicher Aufarbeitung das gewünschte Produkt als Diastereomerengemisch (9:1), welches chromatographisch aufgetrennt wird.
Ausbeute: 30 mg (8%) des weniger polaren Epimers von tert-Butyl (4S,5R)-4-(cyclohexylmethyl) -5-[(1S oder R,2S oder R)-1,2-dihydroxycyclohexyl]-2,2-dimethyl -3-oxazolidincarboxylat als Oel, $R_F$:0.3 (4:1-Gemisch von Toluol und Essigester als Laufmittel) und 330 mg (89%) des polareren Epimers tert-Butyl (4S,5R)-4-(cyclohexylmethyl)-5-[(1R oder S,2R oder S)-1,2-dihydroxycyclohexyl]-2,2-dimethyl -3-oxazolidincarboxylat als Schaum, $R_F$:0.28 (4:1-Gemisch von Toluol und Essigester als Laufmittel).

310 mg (0.753 mMol) tert-Butyl (4S,5R)-4-(cyclohexylmethyl)-5-[(1S oder R,2S oder R)-1,2-dihydroxycyclohexyl]-2,2-dimethyl -3-oxazolidincarboxylat (bzw. tert-Butyl (4S,5R)-4-(cyclohexylmethyl)-5-[-(1R oder S,2R oder S)-1,2-dihydroxycyclohexyl]-2,2-dimethyl -3-oxazolidincarboxylat) löst man in 8 ml Methanol, gibt 4 ml 2N Salzsäure zu und rührt 22 Stunden bei Raumtemperatur. Dann dampft man das Reaktionsgemisch unter vermindertem Druck ein, gibt noch zweimal Toluol zu und dampft jeweils wieder unter vermindertem Druck ein. Auf diese Weise erhält man (1R oder S,2R oder S)-1-[(1R,2S)-1-Amino-3-cyclohexyl -1-hydroxypropyl]-1,2-cyclohexandiol als amorphen Festkörper, $R_F$:0.15 (65:10:1-Gemisch von Methylenchlorid, Methanol und Ammoniak als Laufmittel).

Beispiel A

Eine sterilfiltrierte, wässrige Lösung von (R oder S)-N-[(1S,2R,3S)-1-(Cyclohexylmethyl) -3-cyclopropyl-2,3-dihydroxypropyl]-8-propyl -α,6-bis(3-pyridyl)-s-triazolo[4,3-a]pyrazin -3-acetamid wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| (R oder S)-N-[(1S,2R,3S)-1-(Cyclohexylmethyl) -3-cyclopropyl-2,3-dihydroxypropyl]-8-propyl -α,6-bis(3-pyridyl)--s-triazolo[4,3-a]pyrazin -3-acetamid | 3,0 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel B

Man löst 5 mg (R oder S)-N-[(1S,2R,3S)-1-(Cyclohexylmethyl) -3-cyclopropyl-2,3-dihydroxypropyl]-8-

propyl -α,6-bis(3-pyridyl)-s-triazolo[4,3-a]pyrazin -3-acetamid in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel C

In einer Mischung von 3,5 ml Myglyol 812 und 0,08 g Benzylalkohol werden 500 mg fein gemahlenes (R oder S)-N-[(1S,2R,3S)-1-(Cyclohexylmethyl)-3-cyclopropyl-2,3-dihydroxypropyl]-8-propyl -α,6-bis(3-pyridyl)-s-triazolo[4,3-a]pyrazin -3-acetamld suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

Beispiel D

Wenn man nach den in den Beispielen A-C beschriebenen Verfahren arbeitet, können aus den folgenden, ebenfalls bevorzugten Verbindungen entsprechende galenische Präparate hergestellt werden:

(R oder S)-N-[(1S,2R,3S)-1-(Cyclohexylmethyl)-3-cyclopropyl -2,3-dihydroxypropyl]-8-isopropyl-α-(imidazol -4-ylmethyl)-6-(3-pyridyl)-s-triazolo[4,3-a]pyrazin -3-acetamid;

(R oder S)-N-[(1S,2R,3S)-1-(Cyclohexylmethyl)-3-cyclohexyl -2,3-dihydroxypropyl]-α-(imidazol-4-ylmethyl) -8-isobutyl-6-(3-pyridyl)-s-triazolo[4,3-a]pyrazin-3-acetat;

(R oder S)-N-[(1S,2R,3S)-1-(Cyclohexylmethyl)-2,3-dihydroxy -5-methylhexyl]-8-propyl-6-(3-pyridyl) -α-(3-pyridylmethyl)-s-triazolo[4,3-a]pyrazin-3-acetamid;

(S oder R)-N-[(1S,2R,3S)-1-(Cyclohexylmethyl)-3-cyclopropyl -2,3-dihydroxypropyl]-8-[(RS)-1-methylpropyl] -α-(3-pyridylmethyl)-6-(3-pyridyl) -s-triazolo[4,3-a]pyrazin-3-acetamid und

N-[(1S,2R,3S)-1-(Cyclohexylmethyl-3-cyclopropyl-2,3-dihydroxypropyl]-8-[(RS)-1-methylpropyl]-6-(3-pyridyl)-s-triazolo[4,3-a]pyrazin-3-carboxamid.

**Patentansprüche**

1. Aminosäurederivate der allgemeinen Formel

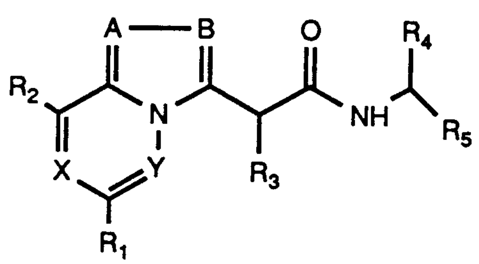

I

worin einer von A und B ein Stickstoffatom und der andere -CH- oder beide ein Stickstoffatom, einer von X und Y ein Stickstoffatom und der andere -CH- oder beide -CH-, $R^1$ Phenyl, Pyridyl oder Thienyl, $R^2$ Alkyl oder Arylalkyl, $R^3$ Wasserstoff, Alkyl, Imidazol-2-ylmethyl, Imidazol-4-ylmethyl, Pyridylmethyl, Pyrazol-3-ylmethyl, Thien-2-ylmethyl, Thiazol-4-ylmethyl, Alkylthiomethyl, Carbamoylmethyl, Carbamoyläthyl oder Benzyl, $R^4$ Cyclohexylmethyl, Benzyl oder Isobutyl und $R^5$ eine der Gruppen

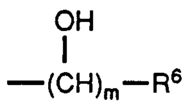

(a)

und

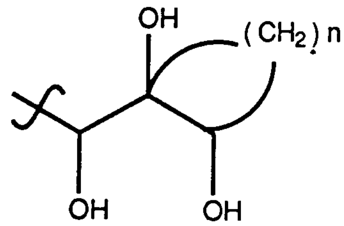

(b)

bedeuten, worin $R^6$ Cycloalkyl, Alkyl, Alkenyl oder Arylalkyl, m die Zahl 2 oder 3 und n die Zahl 3 oder 4 bedeuten,
in Form von optisch reinen Diastereomeren,
Diastereomerengemischen, diastereomeren Racematen oder Gemischen von diastereomeren Racematen sowie pharmazeutisch verwendbare Salze dieser Verbindungen.

**2.** Verbindungen gemäss Anspruch 1, worin A und B je ein Stickstoffatom bedeuten.

**3.** Verbindungen gemäss Anspruch 1 oder 2, worin X ein Stickstoffatom und Y -CH- bedeuten.

**4.** Verbindungen gemäss einem der Ansprüche 1-3, worin $R^1$ Pyridyl, vorzugsweise 3-Pyridyl, bedeutet.

**5.** Verbindungen gemäss einem der Ansprüche 1-4, worin $R^2$ Alkyl oder Phenylalkyl, vorzugsweise Propyl, Isopropyl, Isobutyl, 1-Methylpropyl oder Benzyl, bedeutet.

**6.** Verbindungen gemäss einem der Ansprüche 1-5, worin $R^3$ Wasserstoff, Imidazol-2-ylmethyl, Imidazol-4-ylmethyl oder Pyridylmethyl, vorzugsweise Imidazol-4-ylmethyl oder Pyridyl-3-methyl, bedeutet.

**7.** Verbindungen gemäss einem der Ansprüche 1-6, worin $R^4$ Cyclohexylmethyl bedeutet.

**8.** Verbindungen gemäss einem der Ansprühe 1-7, worin $R^5$ die Gruppe (a) bedeutet.

**9.** Verbindungen gemäss Anspruch 8, worin m die Zahl 2 und $R^6$ Cycloalkyl bedeuten.

**10.** Verbindungen gemäss einem der Ansprüche 1-9, worin A, B und X je ein Stickstoffatom, Y -CH-, $R^1$ 3-Pyridyl, $R^2$ Propyl, Isopropyl, Isobutyl, 1-Methylpropyl oder Benzyl, $R^3$ Imidazol-4-ylmethyl oder Pyridyl-3-methyl, $R^4$ Cyclohexylmethyl und $R^5$ die Gruppe (a) bedeuten, worin m die Zahl 2 und $R^6$ Cycloalkyl bedeuten.

**11.** (R oder S)-N-[(1S,2R,3S)-1-(Cyclohexylmethyl) -3-cyclopropyl-2,3-dihydroxypropyl]-8-propyl -α,6-bis(3-pyridyl)-s-triazolo[4,3-a]pyrazin -3-acetamid.

**12.** (R oder S)-N-[(1S,2R,3S)-1-(Cyclohexylmethyl)-3-cyclopropyl -2,3-dihydroxypropyl]-8-isopropyl-α-(imidazol -4-ylmethyl)-6-(3-pyridyl)-s-triazolo[4,3-a]pyrazin -3-acetamid.

**13.** (R oder S)-N-[(1S,2R,3S)-1-(Cyclohexylmethyl)-3-cyclohexyl -2,3-dihydroxypropyl]-α-(imidazol-4-ylmethyl) -8-isobutyl-6-(3-pyridyl)-s-triazolo[4,3-a]pyrazin-3-acetat.

**14.** (R oder S)-N-[(1S,2R,3S)-1-(Cyclohexylmethyl)-2,3-dihydroxy -5-methylhexyl]-8-propyl-6-(3-pyridyl) -α-(3-pyridylmethyl)-s-triazolo[4,3-a]pyrazin-3-acetamid.

**15.** (S oder R)-N-[(1S,2R,3S)-1-(Cyclohexylmethyl)-3-cyclopropyl -2,3-dihydroxypropyl]-8-[(RS)-1-methylpropyl] -α-(3-pyridylmethyl)-6-(3-pyridyl) -s-triazolo[4,3-a]pyrazin-3-acetamid.

**16.** N-[(1S,2R,3S)-1-(Cyclohexylmethyl-3-cyclopropyl -2,3-dihydroxypropyl]-8-[(RS)-1-methylpropyl] -6-(3-pyridyl)-s-triazolo[4,3-a]pyrazin-3-carboxamid.

**17.** Verbindungen der Formeln

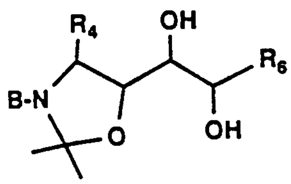

IV

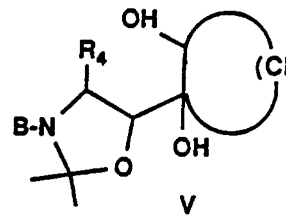

V

und

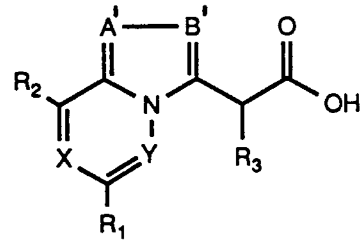

III'

worin B eine Aminoschutzgruppe, $R^4$ Cyclohexylmethyl, Benzyl oder Isobutyl, $R^6$ Cycloalkyl, Alkyl, Alkenyl oder Arylalkyl, n die Zahl 3 oder 4, einer von A' und B' ein Stickstoffatom und der andere -CH- oder beide ein Stickstoffatom und einer von X und Y ein Stickstoffatom und der andere -CH- oder beide -CH-, wobei für den Fall, dass A' und B' je ein Stickstoffatom bedeuten, X -CH- bedeutet, $R^1$ Phenyl, Pyridyl oder Thienyl, $R^2$ Alkyl oder Arylalkyl, $R^3$ Wasserstoff, Alkyl, Imidazol-2-ylmethyl, Imidazol-4-ylmethyl, Pyridylmethyl, Pyrazol-3-ylmethyl, Thien-2-ylmethyl, Thiazol-4-ylmethyl, Alkylthiomethyl, Carbamoylmethyl, Carbamoyläthyl oder Benzyl und $R^5$ eine der Gruppen

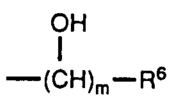

(a)

und

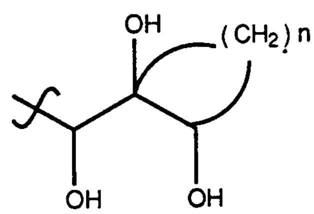

(b)

bedeuten, worin m die Zahl 2 oder 3 bedeutet.

**18.** Aminosäurderivate gemäss einem der Ansprüche 1-16 zur Anwendung als therapeutische Wirkstoffe.

**19.** Aminosäurederivate gemäss einem der Ansprüche 1-16 zur Anwendung bei der Bekämpfung bzw. Verhütung von Bluthochdruck und Herzinsuffizienz.

**20.** Verfahren zur Herstellung einer Verbindung gemäss einem der Ansprüche 1-16, dadurch gekennzeichnet, dass man

a) eine Verbindung der allgemeinen Formel

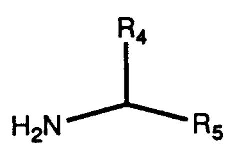

II

worin $R^4$ und $R^5$ die in Anspruch 1 angegebene Bedeutung besitzen,

mit einer Verbindung der allgemeinen Formel

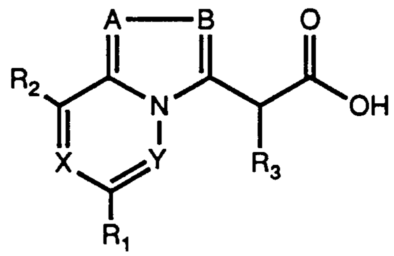

III

worin A, B, X, Y, $R^1$, $R^2$ und $R^3$ die in Anspruch 1 angegebene Bedeutung besitzen, oder einem aktivierten Derivat davon umsetzt, und

b) erwünschtenfalls ein Gemisch diastereomerer Racemate in die diastereomeren Racemate oder optisch reinen Diastereomeren auftrennt, und/oder

c) erwünschtenfalls ein Diastereomerengemisch in die optisch reinen Diastereomeren auftrennt, und/oder

d) erwünschtenfalls eine erhaltene Verbindung in ein pharmazeutisch verwendbares Salz überführt.

**21.** Arzneimittel, enthaltend ein Aminosäurederivat gemäss einem der Ansprüche 1-16 und ein therapeutisch inertes Excipiens.

**22.** Mittel zur Bekämpfung bzw. Verhütung von Bluthochdruck und Herzinsuffizienz, enthaltend ein Aminosäurederivat gemäss einem der Ansprüche 1-16 und ein therapeutisch inertes Excipiens.

**23.** Verwendung eines Aminosäurederivates gemäss einem der Ansprüche 1-16 bei der Bekämpfung bzw. Verhütung von Krankheiten.

**24.** Verwendung eines Aminosäurederivates gemäss einem der Ansprüche 1-16 bei der Bekämpfung bzw. Verhütung von Bluthochdruck und Herzinsuffizienz.

**25.** Verwendung eines Aminosäurederivates gemäss einem der Ansprüche 1-16 zur Herstellung von Mitteln gegen Bluthochdruck und/oder Herzinsuffizienz.

**Patentansprüche für folgenden Vertragsstaat: ES**

**1.** Verfahren zur Herstellung von Aminosäurederivaten der allgemeinen Formel

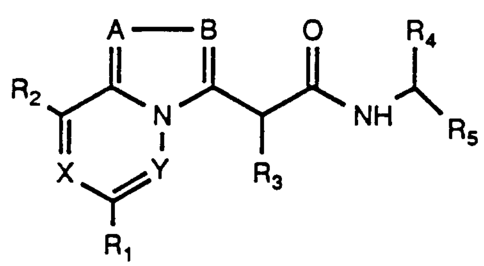

I

worin einer von A und B ein Stickstoffatom und der andere -CH- oder beide ein Stickstoffatom, einer von X und Y ein Stickstoffatom und der andere -CH- oder beide -CH-, $R^1$ Phenyl, Pyridyl oder Thienyl, $R^2$ Alkyl oder Arylalkyl, $R^3$ Wasserstoff, Alkyl, Imidazol-2-ylmethyl, Imidazol-4-ylmethyl, Pyridylmethyl, Pyrazol-3-ylmethyl, Thien-2-ylmethyl, Thiazol-4-ylmethyl, Alkylthiomethyl, Carbamoylmethyl, Carbamoyläthyl oder Benzyl, $R^4$ Cyclohexylmethyl, Benzyl oder Isobutyl und $R^5$ eine der Gruppen

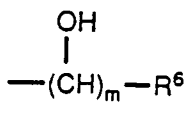

(a)

und

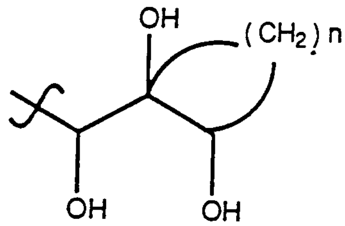

(b)

bedeuten, worin $R^6$ Cycloalkyl, Alkyl, Alkenyl oder Arylalkyl, m die Zahl 2 oder 3 und n die Zahl 3 oder 4 bedeuten,
in Form von optisch reinen Diastereomeren,
Diastereomerengemischen, diastereomeren Racematen oder Gemischen von diastereomeren Racematen sowie pharmazeutisch verwendbaren Salzen dieser Verbindungen, dadurch gekennzeichnet, dass man

a) eine Verbindung der allgemeinen Formel

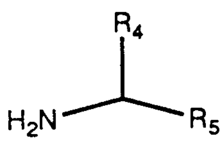

II

worin $R^4$ und $R^5$ die in Anspruch 1 angegebene Bedeutung besitzen,
mit einer Verbindung der allgemeinen Formel

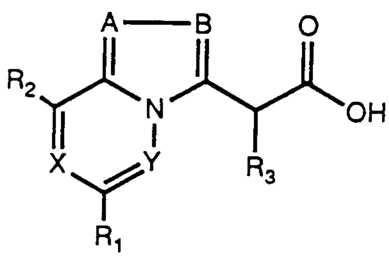

III

worin A, B, X, Y, $R^1$, $R^2$ und $R^3$ die in Anspruch 1 angegebene Bedeutung besitzen,
oder einem aktivierten Derivat davon umsetzt, und

b) erwünschtenfalls ein Gemisch diastereomerer Racemate in die diastereomeren Racemate oder optisch reinen Diastereomeren auftrennt, und/oder

c) erwünschtenfalls ein Diastereomerengemisch in die optisch reinen Diastereomeren auftrennt, und/oder

d) erwünschtenfalls eine erhaltene Verbindung in ein pharmazeutisch verwendbares Salz überführt.

**2.** Verfahren gemäss Anspruch 1, worin A und B je ein Stickstoffatom bedeuten.

**3.** Verfahren gemäss Anspruch 1 oder 2, worin X ein Stickstoffatom und Y -CH- bedeuten.

**4.** Verfahren gemäss einem der Ansprüche 1-3, worin $R^1$ Pyridyl, vorzugsweise 3-Pyridyl, bedeutet.

**5.** Verfahren gemäss einem der Ansprüche 1-4, worin $R^2$ Alkyl oder Phenylalkyl, vorzugsweise Propyl, Isopropyl, Isobutyl, 1-Methylpropyl oder Benzyl, bedeutet.

**6.** Verfahren gemäss einem der Ansprüche 1-5, worin $R^3$ Wasserstoff, Imidazol-2-ylmethyl, Imidazol-4-ylmethyl oder Pyridylmethyl, vorzugsweise Imidazol-4-ylmethyl oder Pyridyl-3-methyl, bedeutet.

7. Verfahren gemäss einem der Ansprüche 1-6, worin $R^4$ Cyclohexylmethyl bedeutet.

8. Verfahren gemäss einem der Ansprühe 1-7, worin $R^5$ die Gruppe (a) bedeutet.

9. Verfahren gemäss Anspruch 8, worin m die Zahl 2 und $R^6$ Cycloalkyl bedeuten.

10. Verfahren gemäss einem der Ansprüche 1-9, worin A, B und X je ein Stickstoffatom, Y -CH-, $R^1$ 3-Pyridyl, $R^2$ Propyl, Isopropyl, Isobutyl, 1-Methylpropyl oder Benzyl, $R^3$ Imidazol-4-ylmethyl oder Pyridyl-3-methyl, $R^4$ Cyclohexylmethyl und $R^5$ die Gruppe (a) bedeuten, worin m die Zahl 2 und $R^6$ Cycloalkyl bedeuten.

11. Verfahren gemäss Anspruch 1, dadurch gekennzeichnet, dass man (R oder S)-N-[(1S,2R,3S)-1-(Cyclohexylmethyl) -3-cyclopropvl-2,3-dihydroxypropyl]-8-propyl -α,6-bis(3-pyridyl)-s-triazolo[4,3-a]-pyrazin -3-acetamid herstellt.

12. Verfahren gemäss Anspruch 1, dadurch gekennzeichnet, dass man (R oder S)-N-[(1S,2R,3S)-1-(Cyclohexylmethyl)-3-cyclopropyl -2,3-dihydroxypropyl]-8-isopropyl-α-(imidazol -4-ylmethyl)-6-(3-pyridyl)-s-triazolo[4,3-a]pyrazin -3-acetamid herstellt.

13. Verfahren gemäss Anspruch 1, dadurch gekennzeichnet, dass man (R oder S)-N-[(1S,2R,3S)-1-(Cyclohexylmethyl)-3-cyclohexyl -2,3-dihydroxypropyl]-α-(imidazol-4-ylmethyl) -8-isobutyl-6-(3-pyridyl)-s-triazolo[4,3-a]pyrazin-3-acetat herstellt.

14. Verfahren gemäss Anspruch 1, dadurch gekennzeichnet, dass man (R oder S)-N-[(1S,2R,3S)-1-(Cyclohexylmethyl)-2,3-dihydroxy -5-methylhexyl]-8-propyl-6-(3-pyridyl) -α-(3-pyridylmethyl)-s-triazolo-[4,3-a]pyrazin-3-acetamid herstellt.

15. Verfahren gemäss Anspruch 1, dadurch gekennzeichnet, dass man (S oder R)-N-[(1S,2R,3S)-1-(Cyclohexylmethyl)-3-cyclopropyl -2,3-dihydroxypropyl]-8-[(RS)-1-methylpropyl] -α-(3-pyridylmethyl)-6-(3-pyridyl) -s-triazolo[4,3-a]pyrazin-3-acetamid herstellt.

16. Verfahren gemäss Anspruch 1, dadurch gekennzeichnet, dass man N-[(1S,2R,3S)-1-(Cyclohexylmethyl-3-cyclopropyl -2,3-dihydroxypropyl]-8-[(RS)-1-methylpropyl] -6-(3-pyridyl)-s-triazolo[4,3-a]pyrazin-3-carboxamid herstellt.

17. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

18. Verwendung eines Aminosäurederivates der Formel I in Anspruch 1, in Form eines optisch reinen Diastereomeren, eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.

**Patentansprüche für folgenden Vertragsstaat: GR**

1. Verfahren zur Herstellung von Aminosäurederivaten der allgemeinen Formel

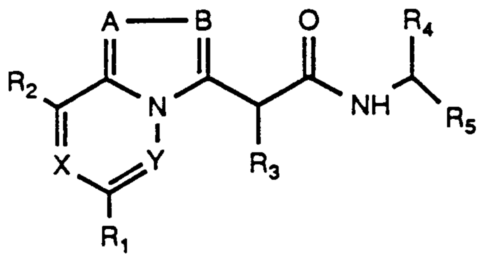

I

worin einer von A und B ein Stickstoffatom und der andere -CH- oder beide ein Stickstoffatom, einer von X und Y ein Stickstoffatom und der andere -CH- oder beide -CH-, $R^1$ Phenyl, Pyridyl oder Thienyl, $R^2$ Alkyl oder Arylalkyl, $R^3$ Wasserstoff, Alkyl, Imidazol-2-ylmethyl, Imidazol-4-ylmethyl, Pyridylmethyl, Pyrazol-3-ylmethyl, Thien-2-ylmethyl, Thiazol-4-ylmethyl, Alkylthiomethyl, Carbamoylmethyl, Carbamoyläthyl oder Benzyl, $R^4$ Cyclohexylmethyl, Benzyl oder Isobutyl und $R^5$ eine der Gruppen

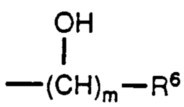

(a)

und

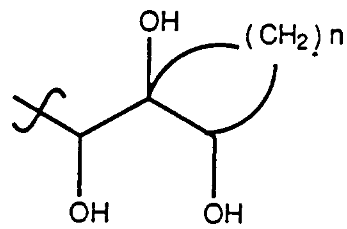

(b)

bedeuten, worin $R^6$ Cycloalkyl, Alkyl, Alkenyl oder Arylalkyl, m die Zahl 2 oder 3 und n die Zahl 3 oder 4 bedeuten,
in Form von optisch reinen Diastereomeren,
Diastereomerengemischen, diastereomeren Racematen oder Gemischen von diastereomeren Racematen sowie pharmazeutisch verwendbaren Salzen dieser Verbindungen, dadurch gekennzeichnet, dass man

a) eine Verbindung der allgemeinen Formel

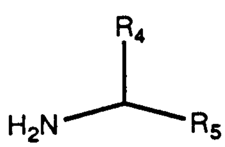

II

worin $R^4$ und $R^5$ die in Anspruch 1 angegebene Bedeutung besitzen,
mit einer Verbindung der allgemeinen Formel

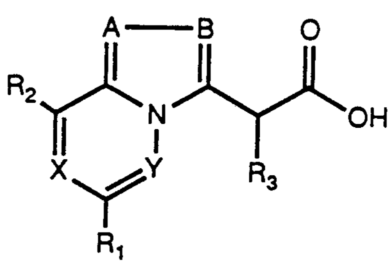

III

worin A, B, X, Y, $R^1$, $R^2$ und $R^3$ die in Anspruch 1 angegebene Bedeutung besitzen,
oder einem aktivierten Derivat davon umsetzt, und

b) erwünschtenfalls ein Gemisch diastereomerer Racemate in die diastereomeren Racemate oder

optisch reinen Diastereomeren auftrennt, und/oder

c) erwünschtenfalls ein Diastereomerengemisch in die optisch reinen Diastereomeren auftrennt, und/oder

d) erwünschtenfalls eine erhaltene Verbindung in ein pharmazeutisch verwendbares Salz überführt.

**2.** Verfahren gemäss Anspruch 1, worin A und B je ein Stickstoffatom bedeuten.

**3.** Verfahren gemäss Anspruch 1 oder 2, worin X ein Stickstoffatom und Y -CH- bedeuten.

**4.** Verfahren gemäss einem der Ansprüche 1-3, worin $R^1$ Pyridyl, vorzugsweise 3-Pyridyl, bedeutet.

**5.** Verfahren gemäss einem der Ansprüche 1-4, worin $R^2$ Alkyl oder Phenylalkyl, vorzugsweise Propyl, Isopropyl, Isobutyl, 1-Methylpropyl oder Benzyl, bedeutet.

**6.** Verfahren gemäss einem der Ansprüche 1-5, worin $R^3$ Wasserstoff, Imidazol-2-ylmethyl, Imidazol-4-ylmethyl oder Pyridylmethyl, vorzugsweise Imidazol-4-ylmethyl oder Pyridyl-3-methyl, bedeutet.

**7.** Verfahren gemäss einem der Ansprüche 1-6, worin $R^4$ Cyclohexylmethyl bedeutet.

**8.** Verfahren gemäss einem der Ansprühe 1-7, worin $R^5$ die Gruppe (a) bedeutet.

**9.** Verfahren gemäss Anspruch 8, worin m die Zahl 2 und $R^6$ Cycloalkyl bedeuten.

**10.** Verfahren gemäss einem der Ansprüche 1-9, worin A, B und X je ein Stickstoffatom, Y -CH-, $R^1$ 3-Pyridyl, $R^2$ Propyl, Isopropyl, Isobutyl, 1-Methylpropyl oder Benzyl, $R^3$ Imidazol-4-ylmethyl oder Pyridyl-3-methyl, $R^4$ Cyclohexylmethyl und $R^5$ die Gruppe (a) bedeuten, worin m die Zahl 2 und $R^6$ Cycloalkyl bedeuten.

**11.** Verfahren gemäss Anspruch 1, dadurch gekennzeichnet, dass man (R oder S)-N-[(1S,2R,3S)-1-(Cyclohexylmethyl) -3-cyclopropyl-2,3-dihydroxypropyl]-8-propyl -α,6-bis(3-pyridyl)-s-triazolo[4,3-a]-pyrazin -3-acetamid herstellt.

**12.** Verfahren gemäss Anspruch 1, dadurch gekennzeichnet, dass man (R oder S)-N-[(1S,2R,3S)-1-(Cyclohexylmethyl)-3-cyclopropyl -2,3-dihydroxypropyl]-8-isopropyl-α-(imidazol -4-ylmethyl)-6-(3-pyridyl)-s-triazolo[4,3-a]pyrazin -3-acetamid herstellt.

**13.** Verfahren gemäss Anspruch 1, dadurch gekennzeichnet, dass man (R oder S)-N-[(1S,2R,3S)-1-(Cyclohexylmethyl)-3-cyclohexyl -2,3-dihydroxypropyl]-α-(imidazol-4-ylmethyl) -8-isobutyl-6-(3-pyridyl)-s-triazolo[4,3-a]pyrazin-3-acetat herstellt.

**14.** Verfahren gemäss Anspruch 1, dadurch gekennzeichnet, dass man (R oder S)-N-[(1S,2R,3S)-1-(Cyclohexylmethyl)-2,3-dihydroxy -5-methylhexyl]-8-propyl-6-(3-pyridyl) -α-(3-pyridylmethyl)-s-triazolo-[4,3-a]pyrazin-3-acetamid herstellt.

**15.** Verfahren gemäss Anspruch 1, dadurch gekennzeichnet, dass man (S oder R)-N-[(1S,2R,3S)-1-(Cyclohexylmethyl)-3-cyclopropyl -2,3-dihydroxypropyl]-8-[(RS)-1-methylpropyl] -α-(3-pyridylmethyl)-6-(3-pyridyl) -s-triazolo[4,3-a]pyrazin-3-acetamid herstellt.

**16.** Verfahren gemäss Anspruch 1, dadurch gekennzeichnet, dass man N-[(1S,2R,3S)-1-(Cyclohexylmethyl-3-cyclopropyl -2,3-dihydroxypropyl]-8-[(RS)-1-methylpropyl] -6-(3-pyridyl)-s-triazolo[4,3-a]pyrazin-3-carboxamid herstellt.

**17.** Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

**18.** Verwendung eines Aminosäurederivates der Formel I in Anspruch 1, in Form eines optisch reinen Diastereomeren, eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.

**19.** Verbindungen der Formeln

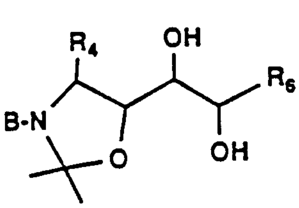

IV ,

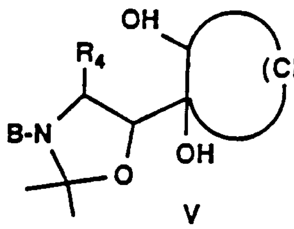

V

und

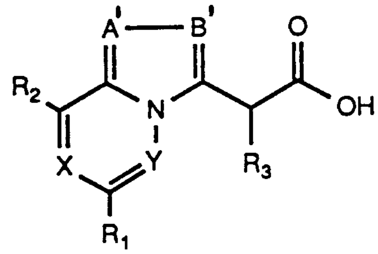

III'

worin B eine Aminoschutzgruppe, $R^4$ Cyclohexylmethyl, Benzyl oder Isobutyl, $R^6$ Cycloalkyl, Alkyl, Alkenyl oder Arylalkyl, n die Zahl 3 oder 4, einer von A' und B' ein Stickstoffatom und der andere -CH- oder beide ein Stickstoffatom und einer von X und Y ein Stickstoffatom und der andere -CH- oder beide -CH-, wobei für den Fall, dass A' und B' je ein Stickstoffatom bedeuten, X -CH- bedeutet, $R^1$ Phenyl, Pyridyl oder Thienyl, $R^2$ Alkyl oder Arylalkyl, $R^3$ Wasserstoff, Alkyl, Imidazol-2-ylmethyl, Imidazol-4-ylmethyl, Pyridylmethyl, Pyrazol-3-ylmethyl, Thien-2-ylmethyl, Thiazol-4-ylmethyl, Alkylthiomethyl, Carbamoylmethyl, Carbamoyläthyl oder Benzyl und $R^5$ eine der Gruppen

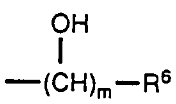

(a)

und

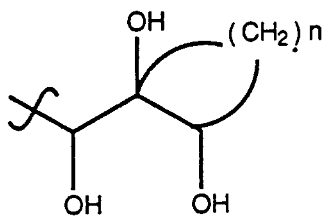

(b)

bedeuten, worin m die Zahl 2 oder 3 bedeutet.